# EUROPEAN PATENT APPLICATION

(11) **EP 4 740 856 A2**
(43) Date of publication of application: **13.05.2026**
(21) Application number: 25209755.5
(22) Date of filing: 20.10.2025
(51) Int. Cl.: A61B 5/145, A61B 5/00

(54) **APPLICATOR AND APPLICATOR ASSEMBLY**

(30) Priority: 07.11.2024 KR 20240157183; 18.02.2025 KR 20250020605
(71) Applicant: i-Sens, Inc., Seoul 06646 (KR)
(72) Inventor: CHOI, Hyun Ho, 06646 Seoul (KR); SEONG, Yul Min, 06646 Seoul (KR); CHAE, Kyung Chul, 06646 Seoul (KR)
(74) Representative: Isarpatent

(57) **Abstract**

According to one aspect of the present invention, an applicator and an applicator assembly capable of stably attaching a wearable unit to skin while inserting a transcutaneous sensor into an accurate subcutaneous position may be provided.

## Description

### CROSS REFERENCE TO RELATED APPLICATION

The present application claims priority to Korean Patent Application No. 10-2024-0157183, filed November 07, 2024, and Korean Patent Application No. 10-2025-0020605, filed February 18, 2025, the entire contents of which are hereby incorporated by reference in its entirety.

### BACKGROUND OF THE INVENTION

### Field of the Invention

The present invention relates to an applicator and an applicator assembly, and more specifically, relates to an applicator and an applicator assembly for inserting a transcutaneous sensor that detects biometric information into subcutaneous tissue.

### Description of the Related Art

Chronic diseases such as diabetes mellitus require continuous management. Diabetes mellitus is characterized by having almost no subjective symptoms in an early stage, but when diabetes mellitus progresses, characteristic symptoms such as polydipsia, polyphagia, polyuria, weight loss, general malaise, skin pruritus, cases in which wounds on hands and feet do not heal and last long appear, and the like. When diabetes mellitus progresses further, complications may occur which advance to visual impairment, hypertension, nephropathy, stroke, periodontal disease, muscle cramps and neuralgia, gangrene, and the like. In order to diagnose such diabetes mellitus and to manage such that progression to complications does not occur, systematic blood glucose measurement and treatment need to be performed in parallel.

Patients with diabetes mellitus or persons in whom glucose levels at a reference value or more is detected in blood although progression to diabetes mellitus has not occurred, require continuous and periodic blood glucose measurement for management of diabetes mellitus or prevention of progression to diabetes mellitus.

Currently, a blood sampling method in which blood is collected from a position such as a fingertip and the like, and blood glucose is measured in a unit of a single time constitutes a mainstream of blood glucose measurement methods. However, in the case of the blood sampling blood glucose measurement method, due to pain accompanied at the time of blood sampling, inconvenience generated therefrom, and the like, there is a limitation in continuous and periodic blood glucose measurement.

Recently, in order to overcome the limitation of the blood sampling blood glucose measurement method, a blood glucose measurement system which measures blood glucose continuously and periodically by inserting a transcutaneous sensor into subcutaneous tissue has been developed and is being used. The blood glucose measurement system using the transcutaneous sensor is generally configured to include a wearable unit provided to be attachable to a human body including the transcutaneous sensor, an applicator which is provided to be coupled to the wearable unit in advance so as to attach the wearable unit to the human body at the same time as inserting the transcutaneous sensor into the subcutaneous tissue, and a receiving unit which processes information received from the wearable unit.

Meanwhile, in terms of hygiene, infection prevention, and the like, the wearable unit is generally provided to be discarded after a single wearing, and the applicator is also generally provided to be discarded after a single firing. That is, when the transcutaneous sensor is not inserted into an accurate subcutaneous position or the wearable unit is not normally attached to the skin, the corresponding wearable unit and the applicator need to each be discarded regardless of their respective usage lifetimes. Therefore, in order to improve the convenience of use and economic efficiency of the blood glucose measurement system using the transcutaneous sensor, there is a need for an applicator which may insert the transcutaneous sensor into an accurate subcutaneous position and may also attach the wearable unit stably to the skin.

### SUMMARY OF THE INVENTION

One aspect of the present invention is to provide an applicator and an applicator assembly capable of stably attaching a wearable unit to skin while inserting a transcutaneous sensor into an accurate subcutaneous position.

The object of the present invention is not limited to the above description. A person with ordinary skill in the art to which the present invention pertains will have no difficulty in readily understanding additional objects of the present invention from the overall contents of the present specification.

An applicator, according to one aspect of the present invention, may comprise: a body housing in which a movement space is formed along a first direction; a sensor unit carrier provided to be movable along the movement space and comprising a fixing groove formed to be recessed on one surface thereof; a handle housing coupled to the body housing and configured such that by at least a portion thereof is in contact with the sensor unit carrier to move together with the sensor unit carrier; and a fixing portion disposed on the body housing so as to transitionally move between a fixing position in which one end portion thereof is inserted into the fixing groove and a release position in which the one end portion is disengaged from the fixing groove.

Movement of the sensor unit carrier may be restricted by the fixing portion positioned at the fixing position, and the movement restriction of the sensor unit carrier may be released as the fixing portion moves to the release position.

The fixing portion may move from the fixing position to the release position in a process of coupling the handle housing to the body housing.

The fixing portion may comprise: a support disposed to extend along a direction parallel to the first direction and provided to be torsionally deformable; a fixing protrusion portion provided to protrude toward the movement space from one surface of the support facing the movement space; and a pressing movement portion connected to the other surface opposite to the one surface of the support facing the fixing protrusion portion.

The handle housing may comprise a push arm extending toward the pressing movement portion from an inner one end of the handle housing, and during coupling of the handle housing to the body housing, a leading end portion of the push arm may press the pressing movement portion so that the fixing portion positioned at the fixing position transitionally moves to the release position.

The pressing movement portion may comprise a pressing movement wing portion provided at a position corresponding to the leading end portion of the push arm WED and the pressing movement wing portion may be provided with a pressing movement inclined surface having an inclination becoming adjacent to the sensor unit carrier along the first direction.

The push arm may comprises a wing portion accommodation hole formed to extend along the first direction in a shape penetrating the push arm, and after the fixing portion moves to the release position, at least one end of the pressing movement wing portion may be disposed in an interior of the wing portion accommodation hole.

The push arm may further comprise a barrier provided on the leading end portion of the push arm adjacent to the fixing portion so as to close one end of the wing portion accommodation hole, and wherein one end of the pressing movement wing portion introduced into the interior of the wing portion accommodation hole contacts with the barrier, thereby restricting a return to an initial position of the handle housing moved in the first direction.

As the leading end portion of the push arm presses the pressing movement inclined surface in the first direction, the support may be torsionally deformed in a direction in which one end of the support to which the fixing protrusion portion and the pressing movement portion are connected moves away from the movement space.

The body housing may further comprise a column disposed to partition the movement space in an interior of the body housing, and the support may be formed by incision of one side wall of the column.

The support may comprises to be divided by a first incision groove and a second incision groove formed adjacent so as to at least partially incise the one side wall of the column.

An applicator assembly, according to one aspect of the present invention, may comprise: a body housing in which a movement space is formed along a first direction, and in which a transmission unit is separably fixed and disposed at one end thereof; a sensor unit carrier comprising a fixing groove formed to be recessed on one side surface thereof, and configured to move in the movement space together with a sensor unit including a transcutaneous sensor member so as to deliver the sensor unit to the transmission unit; a handle housing coupled to the body housing so as to be relatively movable with respect to the body housing and configured to move from a first position to a second position together with the sensor unit carrier by pressing of a user; and a fixing portion disposed on the body housing so as to transitionally move from a fixing position in which one end portion thereof is inserted into the fixing groove to a release position in which the one end portion is disengaged from the fixing groove, during a process of connecting the handle housing to the body housing or during a process in which the handle housing moves from the first position to the second position.

The handle housing may comprise a push arm extending toward the fixing portion from an inner one end of the handle housing, and a leading end portion of the push arm may press the fixing portion so that the fixing portion positioned at the fixing position moves to the release position.

When the handle housing is positioned at the first position, the leading end portion of the push arm may be positioned at a position spaced apart from one end of the fixing portion or positioned at a position in contact with one end of the fixing portion, and as the handle housing moves to the second position, the leading end portion of the push arm may press one end of the fixing portion so that the fixing portion moves to the release position.

The first position may be a position of the handle housing when the sensor unit carrier is positioned at the initial position after completion of assembly of the applicator assembly, and the second position may be a position of the handle housing after the sensor unit carrier moves to the insertion position and the sensor unit is delivered to the transmission unit.

According to one aspect of the present invention, an applicator and an applicator assembly capable of stably attaching a wearable unit to skin while inserting a transcutaneous sensor into an accurate subcutaneous position may be provided.

The effects of the present invention are not limited to the above description, and may include matters that can be reasonably inferred from the following description by a person with ordinary skill in the art to which the present invention pertains.

### BRIEF DESCRIPTION OF THE DRAWINGS

FIG. 1 is a perspective view illustrating an exemplary embodiment of an applicator assembly.
FIG. 2 is a perspective view illustrating an exemplary embodiment of the applicator assembly with a cap removed.
FIG. 3 is a perspective view illustrating an exemplary embodiment of a wearable unit.
FIG. 4 is a conceptual view illustrating an exemplary embodiment of the wearable unit attached to a human body and a remote terminal.
FIG. 5 is a perspective view illustrating an exemplary embodiment of a sensor unit in a state in which needles are coupled.
FIG. 6 is a perspective view illustrating an exemplary embodiment of a coupling process of the sensor unit and a transmission unit.
FIGS. 7 and 8 are exploded perspective views illustrating an exemplary embodiment of the applicator assembly with the cap removed.
FIG. 9 is a perspective view illustrating an exemplary embodiment of a handle housing.
FIG. 10 is a bottom view illustrating an exemplary embodiment of the handle housing.
FIG. 11 is a cross-sectional view illustrating the handle housing of FIGS. 9 and 10 cut in an A-A' direction.
FIG. 12 is a perspective view and a partially enlarged view illustrating an exemplary embodiment of a body housing.
FIG. 13 is a bottom view illustrating an exemplary embodiment of the body housing.
FIG. 14 is a plan view illustrating an exemplary embodiment of the body housing.
FIG. 15 is a cross-sectional view illustrating the body housing cut along B-B' of FIG. 14.
FIG. 16 is a partial cross-sectional perspective view illustrating the body housing cut along C-C' of FIG. 14.
FIG. 17 is a perspective view illustrating an exemplary modified example of a fixing portion, and FIG. 18 is a perspective view and a partial cross-sectional view illustrating another exemplary modified example of the fixing portion.
FIGS. 19 and 20 are perspective views illustrating an exemplary embodiment of a sensor unit carrier.
FIG. 21 is a perspective view exemplarily illustrating a coupling relationship of a needle carrier.
FIG. 22 is a perspective view illustrating an exemplary embodiment of the needle carrier.
FIG. 23 is a front view exemplarily illustrating a coupling relationship of the needle carrier and the sensor unit carrier.
FIG. 24 is an exploded perspective view illustrating an exemplary embodiment of the cap.
FIG. 25 is a cross-sectional view illustrating the cap cut along an E-E' direction in FIG. 24.
FIGS. 26 to 28 are partially enlarged cross-sectional views illustrating an exemplary embodiment of the applicator assembly to which the cap is applied.
FIG. 29 is a partial cross-sectional view illustrating an exemplary embodiment of a state in which the sensor unit carrier is coupled to the body housing during an assembly process of the applicator assembly.
FIGS. 30 and 31 are partial cross-sectional views illustrating an exemplary embodiment of a process of coupling the handle housing to the body housing to which the sensor unit carrier is coupled during an assembly process of the applicator assembly.
FIG. 32 is an enlarged cross-sectional view illustrating an exemplary embodiment of a state in which a leading end portion of a fixing protrusion portion is inserted into a fixing groove during an assembly process of the applicator assembly.
FIGS. 33 to 35 are enlarged cross-sectional views illustrating exemplary embodiments regarding an interaction between a push arm and the fixing portion in the assembly and during an operation process of the applicator assembly.
FIGS. 36 to 38 are partial cross-sectional views sequentially illustrating an exemplary embodiment of a state in which the sensor unit carrier moves from an initial position to an insertion position during an operation process of the applicator assembly.
FIG. 39 is a partial cross-sectional view illustrating an exemplary embodiment of a state in which the needle carrier moves to a retracted position during an operation process of the applicator assembly.
FIG. 40 is a partial cross-sectional view illustrating an exemplary embodiment of a state before a bridge is cut during an operation process of the applicator assembly.
FIG. 41 is a partial cross-sectional view illustrating an exemplary embodiment of a state in which the bridge is cut during an operation process of the applicator assembly.
FIG. 42 is a cross-sectional view and a partial enlarged cross-sectional view for explaining an exemplary embodiment regarding a positional relationship between a first movement restriction portion and a second movement restriction portion in a state before operation of the applicator assembly.
FIGS. 43(a) to 41(c) are sequentially illustrated partial enlarged cross-sectional views for explaining an exemplary embodiment regarding a positional relationship between the first movement restriction portion and the second movement restriction portion during an operation process of the applicator assembly.
FIGS. 44 to 46 are sequentially illustrated cross-sectional views for explaining an exemplary embodiment of pressing and pressing release of a grip arm protrusion portion during an operation process of the applicator assembly.
FIGS. 47 and 48 are sequentially illustrated cross-sectional views for explaining an exemplary embodiment of movement constraint and movement constraint release of a transmission unit by a transmission unit support portion during an operation process of the applicator assembly.

### DETAILED DESCRIPTION OF THE INVENTION

Hereinafter, preferred embodiments of an applicator and an applicator assembly according to one aspect of the present invention will be described in more detail with reference to the accompanying drawings. Embodiments of the present invention may be modified into various forms, and the scope of the present invention should not be construed as being limited to the embodiments described below. These embodiments are provided to more specifically describe the present invention to those skilled in the technical field to which the present invention pertains. Accordingly, a shape of each element illustrated in the drawings may be emphasized or exaggerated for a clearer description.

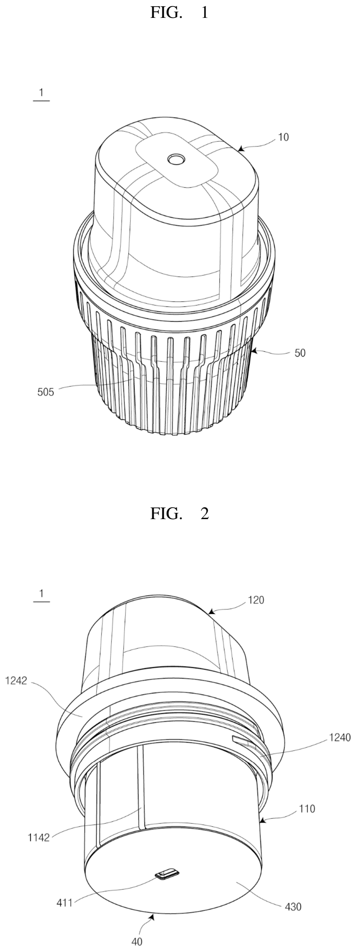

Hereinafter, with reference to FIGS. 1 to 26, the applicator and the applicator assembly will be described in more detail.

### Applicator assembly

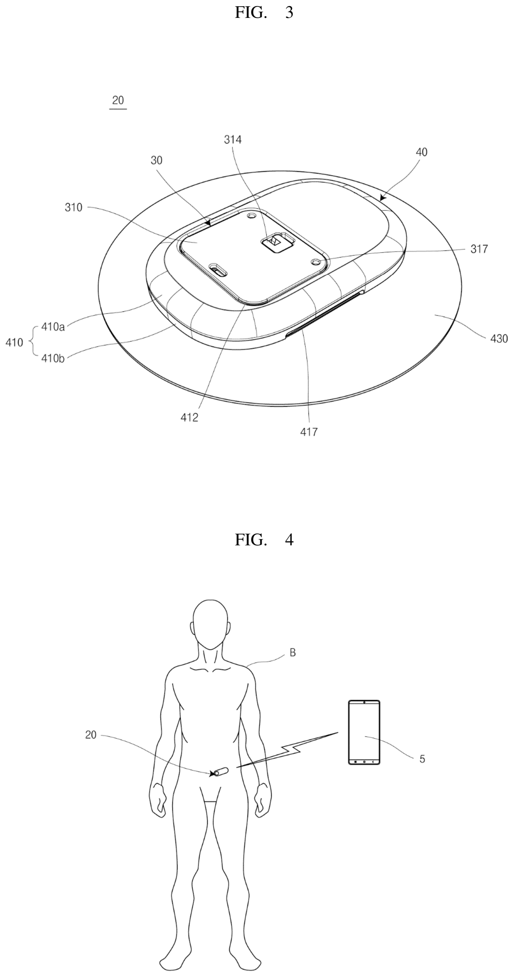

FIG. 1 is a perspective view illustrating an exemplary embodiment of an applicator assembly 1, and FIG. 2 is a perspective view illustrating an exemplary embodiment of the applicator assembly 1 with a cap 50 removed. FIG. 3 is a perspective view illustrating an exemplary embodiment of a wearable unit 20, and FIG. 4 is a conceptual view illustrating an exemplary embodiment of the wearable unit 20 attached to a human body B and a remote terminal 5.

The applicator assembly 1 may include the wearable unit 20 and an applicator 10 that is assembled in advance together with the wearable unit 20 and provided. At one end of the applicator 10, the cap 50 may be separably disposed. By the cap 50 disposed at one end of the applicator 10, arbitrary firing of the applicator 10 may be prevented, and external contaminants or moisture may be prevented from being introduced into an interior of the applicator assembly 1.

The wearable unit 20 may include a transcutaneous sensor member 330 inserted into subcutaneous tissue of the human body B and detecting biometric information. Although the biometric information detected by the transcutaneous sensor member 330 is various, the preferred biometric information detected by the transcutaneous sensor member 330 may be glucose concentration.

The applicator 10 may be used to deliver the wearable unit 20 onto a detection position on skin such that an end portion of the transcutaneous sensor member 330 included in the wearable unit 20 is inserted into the subcutaneous tissue of the human body B. At one end of the wearable unit 20, an adhesive member 430 may be provided, and the wearable unit 20 may be maintained at the detection position for a predetermined period of time by the adhesive member 430. The wearable unit 20 may preferably be provided in the applicator 10 such that an adhesive surface of the adhesive member 430 is exposed to the exterior in a state in which the cap 50 is removed from the applicator assembly 1. The detection position is not limited to a specific position on the human body B, but in terms of convenience in daily life, it may be preferable that the wearable unit 20 is attached on skin of a site of the human body B such as an upper arm, a thigh, an abdomen, or the like.

The wearable unit 20 may be attached to the skin of the human body B, detect biometric information, and wirelessly transmit detected biometric information data to an external terminal 5. A wireless transmission method is not particularly limited, and wireless transmission methods such as Bluetooth (BT), Bluetooth Low Energy (BLE), Near Field Communication (NFC), Radio Frequency Identification (RFID), and the like may be applied. The external terminal 5 is also not particularly limited as long as it is a device capable of receiving and processing data, and a mobile terminal, a dedicated medical device, a PC, a server, and the like may be applied. As a non-limiting example, the wearable unit 20 may continuously or periodically detect glucose concentration of the human body B and transmit glucose concentration data to the external terminal 5.

### Wearable unit

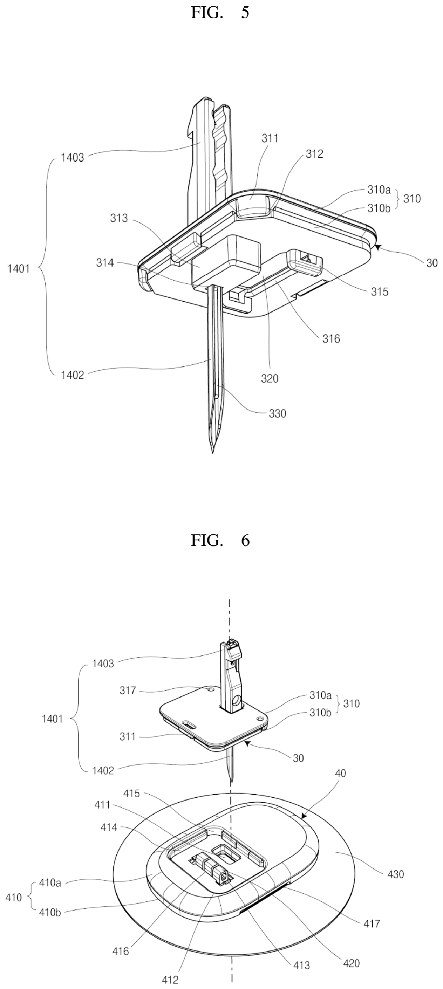

FIG. 5 is a perspective view illustrating an exemplary embodiment of a sensor unit 30 in a state in which needles 1401 are coupled, and FIG. 6 is a perspective view illustrating an exemplary embodiment of a coupling process of the sensor unit 30 and a transmission unit 40.

The wearable unit 20 may include the sensor unit 30 and the transmission unit 40. The wearable unit 20 may be disposed in an interior of the applicator 10 in an integrated form in which the sensor unit 30 and the transmission unit 40 are coupled. In this case, in a firing process of the applicator 10, the wearable unit 20 in the integrated form in which the sensor unit 30 and the transmission unit 40 are coupled may be attached to skin of the human body B.

Meanwhile, it may include a case in which the sensor unit 30 and the transmission unit 40 are disposed in the interior of the applicator 10 in a state separated from each other. In this case, in a process in which the transcutaneous sensor member 330 is inserted into subcutaneous tissue, the sensor unit 30 and the transmission unit 40 may be coupled and disposed at a detection position. It may include all of cases in which the sensor unit 30 and the transmission unit 40 are coupled before the transcutaneous sensor member 330 is inserted into the subcutaneous tissue, or the sensor unit 30 and the transmission unit 40 are coupled simultaneously with insertion of the transcutaneous sensor member 330 into the subcutaneous tissue, or the sensor unit 30 and the transmission unit 40 are coupled after the transcutaneous sensor member 330 is inserted into the subcutaneous tissue. When the wearable unit 20 is disposed in the interior of the applicator 10 in a state in which the sensor unit 30 and the transmission unit 40 are separated, in an insertion process of the transcutaneous sensor member 330 into the subcutaneous tissue, the sensor unit 30 may relatively move with respect to the transmission unit 40, so that coupling of the sensor unit 30 and the transmission unit 40 may be performed. That is, in a state in which the transmission unit 40 is positioned at the detection position, the sensor unit 30 may move toward the transmission unit 40 so that insertion of the transcutaneous sensor member 330 into the subcutaneous tissue may be performed. When the sensor unit 30 moves toward the transmission unit 40 so that insertion of the transcutaneous sensor member 330 into the subcutaneous tissue is performed, compared to the wearable unit 20 provided in an integrated form, even when a small propulsive force is applied to the transcutaneous sensor member 330, the transcutaneous sensor member 330 may be inserted into an accurate position, and pain and discomfort generated in an insertion process of the transcutaneous sensor member 330 into the subcutaneous tissue may be effectively reduced.

The transmission unit 40 may include a transmission unit housing 410 forming an external shape of the transmission unit 40. A first transmission unit housing 410a and a second transmission unit housing 410b may be coupled to each other to form the transmission unit housing 410, and in an interior of the transmission unit housing 410, a battery serving as a power source ,an electronic unit transmitting biometric information data, and the like may be provided. At a coupling portion of the first transmission unit housing 410a and the second transmission unit housing 410b, a sealing portion for preventing external contaminants or moisture from being introduced into the interior of the transmission unit housing 410 may be provided.

At one end of the first transmission unit housing 410a, a seating groove 412 in which the sensor unit 30 is accommodated and coupled may be formed to be recessed toward an inside of the transmission unit housing 410. The seating groove 412 may be formed in a shape recessed toward the inside of the transmission unit housing 410 from one surface of the first transmission unit housing 410a. The seating groove 412 may preferably be formed in a shape corresponding to a sensor unit housing 310 to be described later. In one region of the first transmission unit housing 410a forming a side surface of the seating groove 412, a side surface protrusion portion 414 having a shape protruding toward the seating groove 412 may be provided. The side surface protrusion portion 414 may be divided into a plurality of portions by a division portion 415, and at the division portion 415, a sensor unit housing protrusion portion 311 to be described later may be disposed.

On one surface of the first transmission unit housing 410a forming the seating groove 412, a first connection opening 416 connecting the interior of the transmission unit housing 410 and the exterior may be formed through. A transmission unit connector 420 connected to the electronic unit disposed in the interior of the transmission unit housing 410 may be exposed to the exterior through the first connection opening 416. A shape of the transmission unit connector 420 is not limited to the shape illustrated in the drawings, and as long as the shape is electrically connectable to a connection terminal intended to be connected to the transmission unit connector 420, it is not limited and may be variously modified and applied. It may be preferable that the transmission unit connector 420 is provided in a material capable of conducting electricity, and in terms of contact stability, it may be more preferable that the transmission unit connector 420 is provided in a material or structure having self-elasticity.

At an end portion of the first transmission unit housing 410a forming a boundary with the first connection opening 416, a fastening latch 413 formed to protrude toward an inside of the seating groove 412 may be provided. The fastening latch 413 may be coupled to a fastening ring 315 to be described later, and the fastening latch 413 may be fastened to the fastening ring 315, thereby assisting in maintaining a firm coupled state of the sensor unit 30 and the transmission unit 40.

An insertion hole 411 may be formed in a shape penetrating the first transmission unit housing 410a and the second transmission unit housing 410b from the inside of the seating groove 412. When the transcutaneous sensor member 330 is inserted into subcutaneous tissue, a needle body 1402 and the transcutaneous sensor member 330 may pass through the insertion hole 411 and be inserted into the subcutaneous tissue. After an end portion of the transcutaneous sensor member 330 is inserted into the subcutaneous tissue, the needle body 1402 is discharged from skin through the insertion hole 411, and one end of the transcutaneous sensor member 330 may be maintained in a state inserted into the subcutaneous tissue.

At one end of the second transmission unit housing 410b opposite to one end in which the seating groove 412 is formed, the adhesive member 430 may be provided. By the adhesive member 430 attached to skin, the wearable unit 20 may be maintained at a detection position for a predetermined period of time. The adhesive member 430 may include a first adhesive surface adhered to skin and a second adhesive surface adhered to one end of the second transmission unit housing 410b. For protection of the first adhesive surface before attachment to skin, a protective film may be additionally provided on the first adhesive surface attached to skin. In FIG. 6, the adhesive member 430 provided to have a larger area than one end of the second transmission unit housing 410b is illustrated, but a shape of the adhesive member 430 is not necessarily limited thereto. The adhesive member 430 may be provided to have an area corresponding to one end of the second transmission unit housing 410b, or may be provided to have a smaller area than one end of the second transmission unit housing 410b. The adhesive member 430 may be separately provided so that a user attaches it to the transmission unit housing 410 in an insertion process of the transcutaneous sensor member 330 into subcutaneous tissue, but may include a case in which the adhesive member 430 is provided to maintain a state in which the adhesive member 430 is pre-attached to the transmission unit housing 410 disposed in an interior of the applicator assembly 1.

On a side end of one end side of the second transmission unit housing 410b facing the adhesive member 430, a transmission unit housing groove 417 formed to be recessed toward the inside of the transmission unit housing 410 may be provided. An end portion of a catching portion 1164 provided in a transmission unit support portion 1160 to be described later may be disposed in an interior of the transmission unit housing groove 417, and in this case, the transmission unit 40 may be effectively prevented from being arbitrarily deviated from the applicator 10.

The sensor unit 30 may include a sensor unit housing 310 forming an external shape of the sensor unit 30, and a transcutaneous sensor member 330 disposed in the interior of the sensor unit housing 310 and provided such that one end thereof is exposed to an exterior of the sensor unit housing 310. When the wearable unit 20 is attached to the human body B, one end of the transcutaneous sensor member 330 exposed to the exterior of the sensor unit housing 310 may be maintained in a state inserted into subcutaneous tissue, and biometric information may be detected by the transcutaneous sensor member 330 percutaneously inserted into the subcutaneous tissue.

The sensor unit housing 310 may be formed by coupling a first sensor unit housing 310a and a second sensor unit housing 310b to each other. At a coupling portion of the first sensor unit housing 310a and the second sensor unit housing 310b, a sealing portion for preventing external contaminants or moisture from being introduced into the interior of the sensor unit housing 310 may be provided. At a side end portion of the second sensor unit housing 310b, a plurality of concave portions 312 having shapes recessed from a side surface of the second sensor unit housing 310b may be formed spaced apart at predetermined intervals along a circumferential direction of the second sensor unit housing 310b, and on one surface of the first sensor unit housing 310a facing the second sensor unit housing 310b, a sensor unit housing protrusion portion 311 formed to protrude at positions corresponding to the plurality of concave portions 312 may be provided. The sensor unit housing protrusion portion 311 and the concave portion 312 may be provided to have a shape in which the sensor unit housing protrusion portion 311 is in close contact with the concave portion 312 or the sensor unit housing protrusion portion 311 is capable of pressing the concave portion 312 in a state in which the first sensor unit housing 310a and the second sensor unit housing 310b are coupled. Since the sensor unit housing protrusion portion 311 is maintained in a state of being in close contact with or pressing the concave portion 312 in a state in which the first sensor unit housing 310a and the second sensor unit housing 310b are coupled to each other, the first sensor unit housing 310a and the second sensor unit housing 310b may be maintained in a more firmly coupled state. Meanwhile, the side surface protrusion portion 414 formed in the transmission unit housing 410 may be provided to have a shape being in close contact with a side end of the second sensor unit housing 310b or capable of pressing the side end of the second sensor unit housing 310b in a state in which the sensor unit 30 and the transmission unit 40 are coupled, and by the close contact between the side surface protrusion portion 414 and the second sensor unit housing 310b or by the pressing of the second sensor unit housing 310b by the side surface protrusion portion 414, the sensor unit 30 and the transmission unit 40 may be maintained in a more firmly fixed state. When a coupled state of the sensor unit 30 and the transmission unit 40 is maintained, the sensor unit housing protrusion portion 311 may be disposed in an interior of the division portion 415 formed at a side end of the seating groove 412, and the sensor unit housing protrusion portion 311 may be in close contact with one surface of the first transmission unit housing 410a forming the division portion 415, or one surface of the first transmission unit housing 410a forming the division portion 415 may press the sensor unit housing protrusion portion 311.

At one end of the second sensor unit housing 310b disposed to face the seating groove 412 during coupling of the sensor unit 30 and the transmission unit 40, a second connection opening 316 connecting an interior of the sensor unit housing 310 and the exterior may be formed through. A sensor unit connector 320 connected to the transcutaneous sensor member 330 may be exposed to the exterior through the second connection opening 316. During coupling of the sensor unit 30 and the transmission unit 40, the sensor unit connector 320 and the transmission unit connector 420 may be electrically in contact with each other, and biometric information data detected by the transcutaneous sensor member 330 may be delivered to an electronic unit provided in the transmission unit 40 through the sensor unit connector 320 and the transmission unit connector 420.

At one end of the second sensor unit housing 310b facing the transmission unit 40, a boss 313 formed to protrude in a shape corresponding to the insertion hole 411 may be provided. In the boss 313, a through-hole 314 communicating an interior of the sensor unit housing 310 with the exterior may be formed through, and one end of the transcutaneous sensor member 330 intended to be inserted into subcutaneous tissue may extend from the interior of the sensor unit housing 310 to the exterior of the sensor unit housing 310 through the through-hole 314 and be disposed. The through-hole 314 may be formed to extend through not only the second sensor unit housing 310b but also the first sensor unit housing 310a. The needle body 1402 may be disposed to pass through the through-hole 314 in a state in which the sensor unit 30 and the needle 1401 are coupled. In this case, one end of the transcutaneous sensor member 330 extending to the exterior of the sensor unit housing 310 may be disposed in the exterior of the sensor unit housing 310 in a state accommodated in an interior of the needle body 1402. During coupling of the sensor unit 30 and the transmission unit 40, a circumferential side surface of the boss 313 and an inner surface of the transmission unit housing 410 forming the insertion hole 411 may come into contact with each other in a state of being in close contact. Accordingly, not only may one end of the transcutaneous sensor member 330 be inserted into an accurate position, but also the sensor unit 30 and the transmission unit 40 may be maintained in a more firmly coupled state. Meanwhile, in a state in which the sensor unit 30 and the transmission unit 40 are mutually coupled, the circumferential side surface of the boss 313 and the inner surface of the transmission unit housing 410 forming the insertion hole 411 may be maintained in a state of close contact with each other, and in a state in which the wearable unit 20 is attached to the human body B, it is possible to effectively prevent external contaminants or moisture from being introduced toward the sensor unit connector 320 and the transmission unit connector 420.

At an end portion of the second sensor unit housing 310b forming a boundary with the second connection opening 316, the fastening ring 315 formed to protrude along a direction parallel to a protruding direction of the transcutaneous sensor member 330 may be provided. In a coupling process of the sensor unit 30 and the transmission unit 40, the fastening latch 413 may be fixed to the fastening ring 315, and by mutual coupling of the fastening latch 413 and the fastening ring 315, the sensor unit 30 and the transmission unit 40 may be maintained in a firmly coupled state. In the above, an example is described in which the fastening latch 413 is provided in the first transmission unit housing 410a, and the fastening ring 315 is provided in the second sensor unit housing 310b, but it may include a case in which the fastening latch 413 is provided in the second sensor unit housing 310b, and the fastening ring 315 is provided in the first transmission unit housing 410a. Meanwhile, the fastening means of the sensor unit 30 and the transmission unit 40 are not limited to a latch and a ring, and as long as the fastening means are capable of preventing the sensor unit 30 from being separated from the transmission unit 40 after coupling of the sensor unit 30 and the transmission unit 40, while not interfering with movement of the sensor unit 30 toward the transmission unit 40 in an insertion process of the transcutaneous sensor member 330 into subcutaneous tissue, the fastening means may be variously modified and applied.

On one surface of the first sensor unit housing 310a, a fixing groove 317 may be formed to be recessed, and a leading end portion of a fixing protrusion 1317 formed in a sensor unit carrier 130 to be described later may be inserted into the fixing groove 317 and be disposed. In an insertion process of the transcutaneous sensor member 330 into subcutaneous tissue, the sensor unit 30 may move toward the transmission unit 40 together with the sensor unit carrier 130, and during movement of the sensor unit 30, the leading end portion of the fixing protrusion 1317 may maintain a state of being inserted into the fixing groove 317 so that the sensor unit 30 may be stably supported by the sensor unit carrier 130 and move. After insertion of the transcutaneous sensor member 330 into the subcutaneous tissue, in a process in which a user removes the applicator 10 from skin, the fixing protrusion 1317 may be discharged from the fixing groove 317, and thereby, a fixing relationship between the sensor unit carrier 130 and the sensor unit 30 may be released.

### Applicator

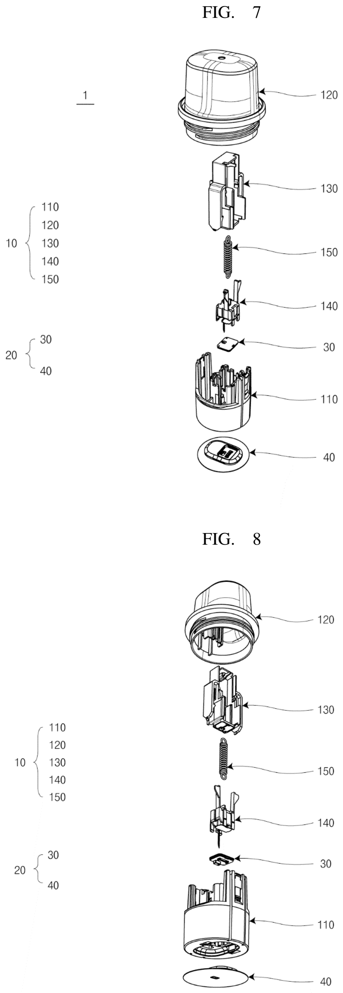

FIGS. 7 and 8 are exploded perspective views illustrating an exemplary embodiment of the applicator assembly 1 with the cap 50 removed. Hereinafter, for convenience of description, a direction generally parallel with an insertion direction of the transcutaneous sensor member 330 is defined as a first direction, and all directions generally perpendicular to the first direction are defined as a second direction, and a specific configuration of the applicator 10 and the applicator assembly 1 is described.

The applicator 10 may be provided to deliver the wearable unit 20 to a detection position on skin. The applicator 10 may include a body housing 110 to which the transmission unit 40 is separably coupled at one end, a handle housing 120 disposed to relatively move with respect to the body housing 110 along the first direction during insertion of the transcutaneous sensor member 330 into subcutaneous tissue, a sensor unit carrier 130 to which the sensor unit 30 is separably coupled at one end and which is disposed to move together with the handle housing 120 along the first direction, a needle carrier 140 provided with the needle body 1402 for insertion of the transcutaneous sensor member 330 into subcutaneous tissue and separably and fixedly disposed to the sensor unit carrier 130, and an elastic member 150 having one end and the other end connected to the sensor unit carrier 130 and the needle carrier 140, respectively, and providing driving force such that the needle body 1402 inserted into the subcutaneous tissue is discharged from the human body B.

The applicator assembly 1 may further include, in addition to the above-described applicator 10, the transmission unit 40 separably and fixedly disposed to one end of the body housing 110, the sensor unit 30 separably disposed to one end of the sensor unit carrier 130, and the cap 50 separably coupled to the handle housing 120 so as to block external exposure of the body housing 110 in which the transmission unit 40 is disposed.

### Handle housing

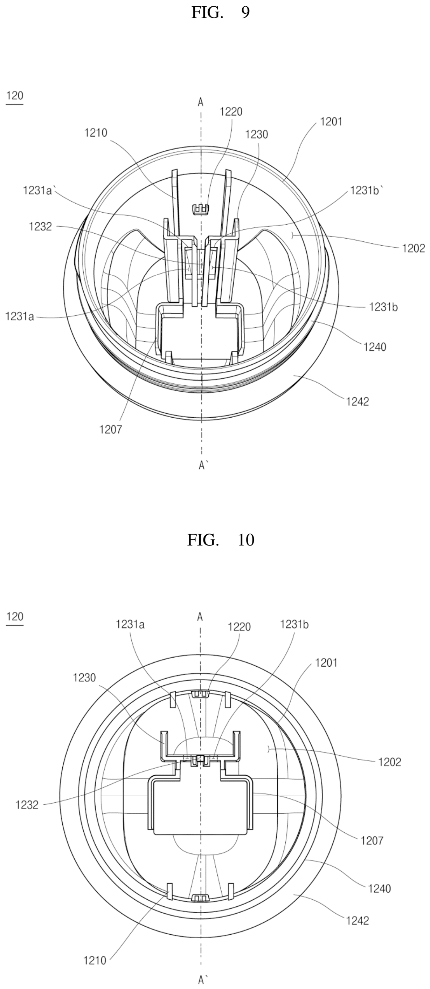

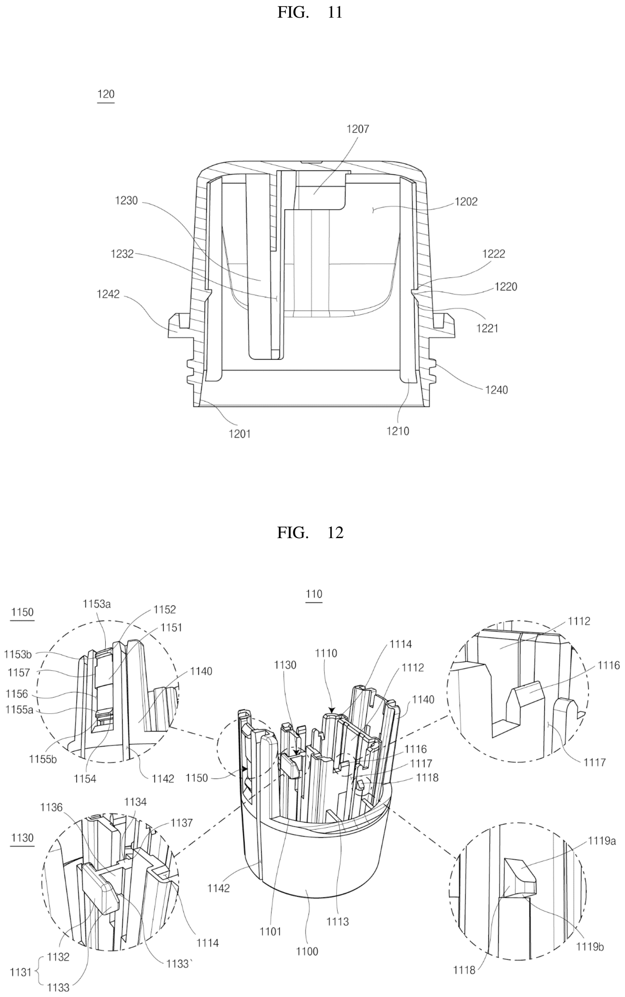

FIGS. 9 and 10 are a perspective view and a bottom view illustrating an exemplary embodiment of the handle housing 120, and FIG. 11 is a cross-sectional view illustrating the handle housing 120 of FIGS. 9 and 10 cut in an A-A' direction.

The handle housing 120 may form an external shape of the applicator 10 together with the body housing 110. The handle housing 120 may be gripped or pressed by a user in an insertion process of the transcutaneous sensor member 330 into subcutaneous tissue. Although FIGS. 9 to 11 illustrate a cup-shaped the handle housing 120, the shape of handle housing 120 is not necessarily limited to the cup shape, and may be variously modified and applied as long as it can achieve the functions described below. However, at one end of the handle housing 120, the cap 50 may be separably coupled by screw-coupling, and therefore, it may be more preferable that one end of the handle housing 120 in which a screw thread for the screw-coupling is formed is provided to have a cylindrical structure.

In an interior of the handle housing 120, a first interior space 1202 may be formed, and the first interior space 1202 may be connected to the exterior by a first opening 1201 formed at one end of the handle housing 120. A push arm 1230 may be formed to extend along the first direction from an inner one surface of the handle housing 120 facing the first opening 1201. The push arm 1230 may be provided to interact with a fixing portion 1130 to be described later, and in an assembly process of the applicator 10, by the push arm 1230 pressing and moving the fixing portion 1130, a temporary movement restriction of the sensor unit carrier 130 by the fixing portion 1130 may be released. A push arm slit 1232 may be formed to extend from a leading end portion of the push arm 1230 in a direction opposite to a protruding direction of the push arm 1230 in a shape dividing an end portion of the push arm 1230. An pressing movement extension portion 1136 provided in the fixing portion 1130 to be described later may be disposed in the push arm slit 1232, so that during an assembly process of the applicator 10 or in an insertion process of the transcutaneous sensor member 330 into subcutaneous tissue, movement interference of the pressing movement extension portion 1136 with respect to the push arm 1230 may be excluded. Although FIGS. 9 to 11 illustrate the push arm 1230 formed symmetrically with respect to the push arm slit 1232, the shape of the push arm 1230 is not necessarily limited thereto, and as long as it is a structure capable of pressing and moving the fixing portion 1130 in the assembly process of the applicator 10, it may be variously modified and applied.

On one side of the push arm slit 1232, a wing portion accommodation hole 1231 penetrating the push arm 1230 in a thickness direction may be formed in a shape extending along the first direction. Meanwhile, the wing portion accommodation hole 1231 may not completely penetrate the push arm 1230 in the thickness direction, but may be formed in a shape recessed from one surface of the push arm 1230, which comes into contact with a pressing movement wing portion 1133 as the handle housing 120 moves in the first direction. The wing portion accommodation hole 1231 may be provided in a shape and at a position corresponding to the pressing movement wing portion 1133, and in case where the pressing movement wing portion 1133 is provided in a pair, the wing portion accommodation hole 1231 may be formed symmetrically with respect to the push arm slit 1232.

The wing portion accommodation hole 1231 may preferably be provided in a shape not extending to an end portion of the push arm 1230, which first comes into contact with the pressing movement wing portion 1133 as the handle housing 120 moves in the first direction. That is, the wing portion accommodation hole 1231 may be formed in the push arm 1230 in a shape extending along the first direction, but may have a shape not extending to an end of the push arm 1230 by a barrier 1231' provided at an end portion of the push arm 1230. The barrier 1231' may be provided in a shape closing the wing portion accommodation hole 1231 in a region on an end portion side of the push arm 1230. In a process in which the handle housing 120 moves along the first direction, at least one end of the pressing movement wing portion 1133 may be introduced into an interior of the wing portion accommodation hole 1231, and one end of the pressing movement wing portion 1133 introduced into the wing portion accommodation hole 1231 may come into contact with the barrier 1231' so that movement of the push arm 1230 in a direction opposite to the first direction may be restricted. That is, by an interaction of the push arm 1230 and a fixing portion 1130, after insertion of the transcutaneous sensor member 330 into the subcutaneous tissue, restoration of the handle housing 120 to an initial position may be restricted.

On an inner one surface of the handle housing 120 facing the first opening 1201, a carrier fixing fence 1207 disposed at a position adjacent to the push arm 1230 may be provided. The carrier fixing fence 1207 may be disposed upright in a shape corresponding to an entire or partial circumferential portion at one end portion side of the sensor unit carrier 130. In an assembly process of the applicator 10, one end portion of the sensor unit carrier 130 is inserted by fitting into the carrier fixing fence 1207, so that the sensor unit carrier 130 and the handle housing 120 may be coupled. Since a circumferential surface on the one end portion side of the sensor unit carrier 130 comes into close contact with and fixed to an inner surface of the carrier fixing fence 1207, the sensor unit carrier 130 may move along the first direction together with the handle housing 120 during insertion of the transcutaneous sensor member 330 into the subcutaneous tissue.

On an inner side surface of the handle housing 120 forming the first interior space 1202, a guide protrusion 1210 extending along the first direction in a shape protruding toward the first interior space 1202 may be provided. A leading end portion of the guide protrusion 1210 may be disposed in an interior of a handle housing guide groove 1142 formed to be recessed on an outer surface of a body housing 110 to be described later, and through interaction between the guide protrusion 1210 and the handle housing guide groove 1142, a movement direction of the handle housing 120 moving in the first direction may be guided. The guide protrusion 1210 may be provided in a pair in a symmetrical shape on the inner side surface of the handle housing 120, and the handle housing guide groove 1142 may also be formed to be recessed at a position corresponding to the guide protrusion 1210 in a shape corresponding to the guide protrusion 1210. Meanwhile, although a case in which the guide protrusion 1210 is formed on the handle housing 120 and the handle housing guide groove 1142 is formed on the body housing 110 has been exemplarily described, cases in which a guide groove is formed on the handle housing and a guide protrusion is formed on the body housing may be included.

A first movement restriction portion 1220 may be formed to protrude toward a central portion of the first interior space 1202 from the inner side surface of the handle housing 120 forming the first interior space 1202 of the handle housing 120. The first movement restriction portion 1220 may interact with a second movement restriction portion 1150 provided in the body housing 110 to be described later. By the interaction between the first movement restriction portion 1220 and the second movement restriction portion 1150, it is possible to restrict the handle housing 120 coupled to the body housing 110 from being arbitrarily deviated from the body housing 110, or to restrict the handle housing 120 from moving in a direction opposite to the first direction after insertion of the transcutaneous sensor member 330 into subcutaneous tissue. The first movement restriction portion 1220 may be provided as a latch structure having a wedge-shaped cross-section provided with an inclined surface 1221 and a support surface 1222, but the shape of the first movement restriction portion 1220 is not necessarily limited thereto, and as long as it is a shape capable of restricting the handle housing 120 from being arbitrarily deviated from the body housing 110, or restricting the handle housing 120 from moving in a direction opposite to the first direction after insertion of the transcutaneous sensor member 330 into the subcutaneous tissue through interaction with the second movement restriction portion 1150, it may be applied without limitation.

On an outer surface of one end portion side of the handle housing 120 where the first opening 1201 is formed, a screw threaded portion 1240 for screw-coupling with the cap 50 may be provided. The screw threaded portion 1240 formed in the handle housing 120 and a screw threaded portion 540 formed in the cap 50 to be described later may be screw-coupled so that the handle housing 120 and the cap 50 may be coupled to each other in a separable manner. On an outer surface of the handle housing 120, a locking protrusion portion 1242 protruding toward the outside from the outer surface of the handle housing 120 and extending along a circumferential direction of the handle housing 120 may be provided. Since the locking protrusion portion 1242 may be provided in a structure capable of contacting a leading end portion of the cap 50, during screw-coupling between the handle housing 120 and the cap 50, it is possible to prevent excessive tightening between the screw threaded portion 1240 of the handle housing 120 and the screw threaded portion 540 of the cap 50. Meanwhile, a structure may be provided in which, in a state where the handle housing 120 and the cap 50 are mutually coupled, the locking protrusion portion 1242 and an end portion of the cap 50 are in close contact, and in this case, it is possible to effectively prevent external contaminants or moisture from being introduced into an interior of the applicator assembly 1. Although screw-coupling by screw threads respectively formed on the handle housing 120 and the cap 50 has been described as one example of a coupling method of the handle housing 120 and the cap 50, the handle housing 120 and the cap 50 may be applied by being modified into various coupling methods such as a fitting coupling method, a fastening method by a latch, and the like.

Although screw-coupling by screw threads respectively formed on the handle housing 120 and the cap 50 has been described as one example of a coupling method of the handle housing 120 and the cap 50, the handle housing 120 and the cap 50 may be applied by being modified into various coupling methods such as a fitting coupling method, a method by a latch, and the like. Meanwhile, a fastening member for coupling of the handle housing 120 and the cap 50 may not only be respectively provided in singular on the handle housing 120 and the cap 50, but also may include a case in which a plurality of fastening members are respectively disposed at positions corresponding to each other on the handle housing 120 and the cap 50.

### Body housing

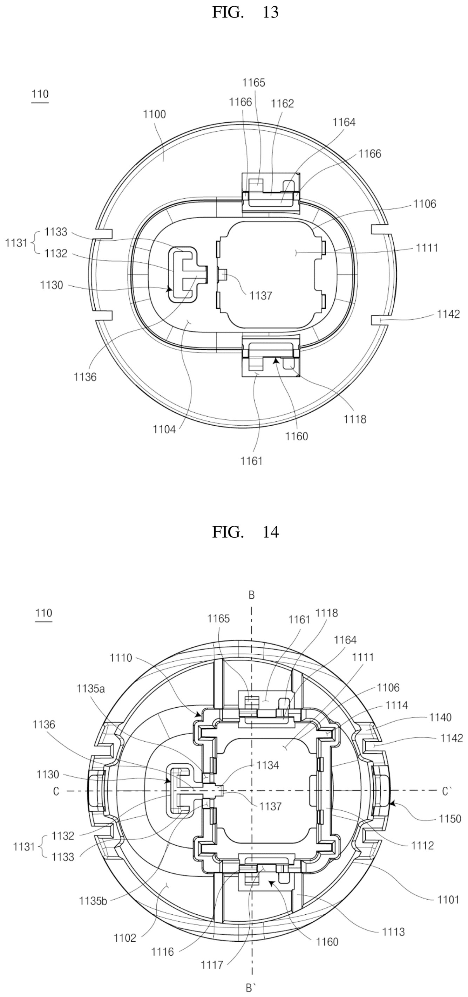

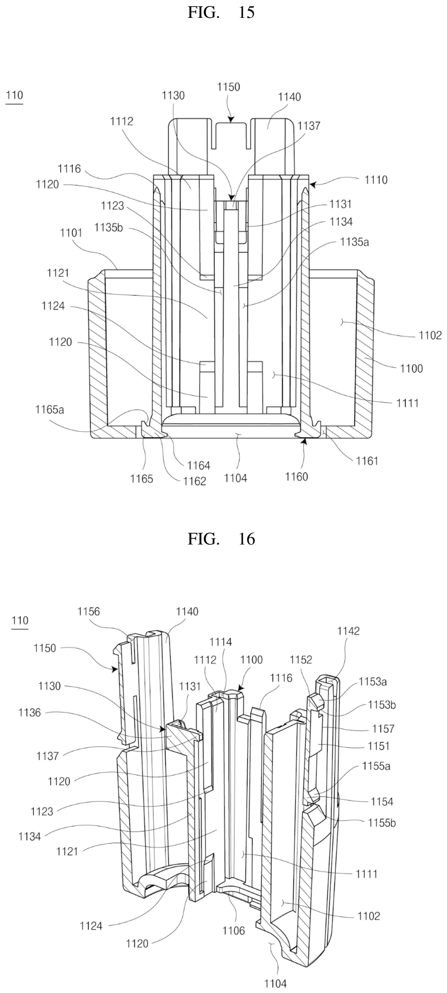

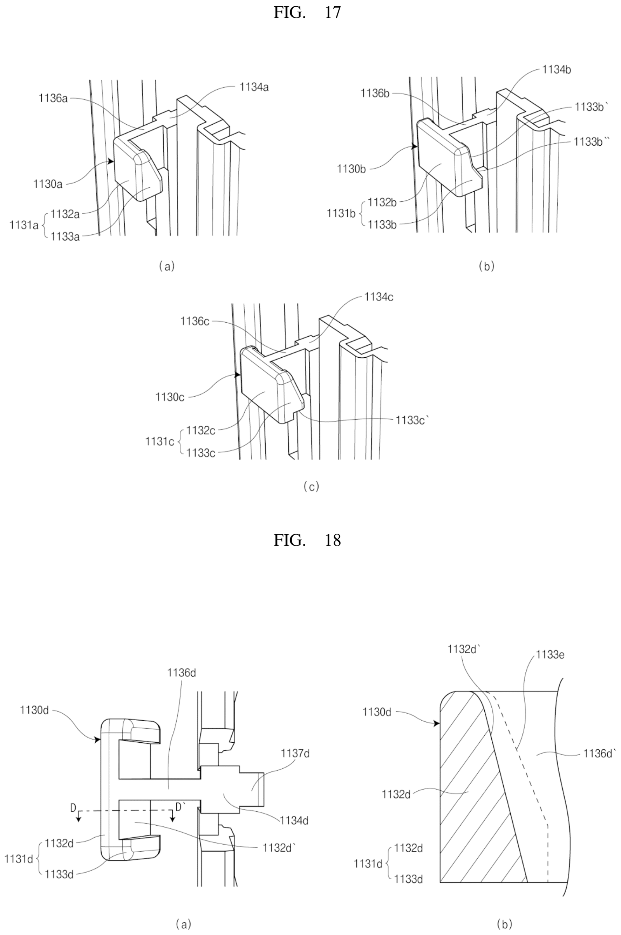

FIG. 12 is a perspective view and a partially enlarged view illustrating an exemplary embodiment of the body housing 110, FIG. 13 is a bottom view illustrating an exemplary embodiment of the body housing 110, and FIG. 14 is a plan view illustrating an exemplary embodiment of the body housing 110. FIG. 15 is a cross-sectional view of the body housing 110 cut along B-B' of FIG. 14, and FIG. 16 is a partial cross-sectional perspective view of the body housing 110 cut along C-C' of FIG. 14. FIGS. 17(a) to 17(c) are perspective views illustrating exemplary modified examples of a fixing portion, FIG. 18(a) is a perspective view illustrating an exemplary modified example of the fixing portion, and FIG. 18(b) is a partial cross-sectional view taken along D-D' of FIG. 18(a).

The body housing 110 may be provided so as to support the sensor unit carrier 130 disposed in an interior of the applicator 10 to guide a movement direction of the sensor unit carrier 130 and to restrict a movement range of the sensor unit carrier 130, and at one end of the body housing 110, the transmission unit 40 for attachment to the human body B may be separably and fixedly disposed.

The body housing 110 may include a body housing body portion 1100 having a circumferential surface of a shape corresponding to the first interior space 1202 of the handle housing 120. In an interior of the body housing body portion 1100, a second interior space 1102 may be provided, and the second interior space 1102 may be connected to the exterior through a second opening 1101 formed at one end of the body housing 110 adjacent to the handle housing 120. When the body housing 110 and the handle housing 120 are assembled to be coupled to each other, the second interior space 1102 formed in the body housing 110 and the first interior space 1202 formed in the handle housing 120 may be connected to each other to form an interior space (not illustrated) of the interior of the applicator 10 separated from the exterior. Meanwhile, the other end side of the body housing 110 facing the one end where the second opening 1101 is formed may be provided in a closed form.

In the interior of the body housing 110, a column 1110 in which a first movement space 1111 is formed to penetrate along the first direction may be disposed upright. The column 1110 may include a plurality of partition walls 1112 extending along a direction opposite to the first direction from an inner surface of the closed other end side of the body housing 110 and disposed upright. The plurality of partition walls 1112 may be provided to surround the first movement space 1111 from a side surface side, and by the plurality of partition walls 1112, the second interior space 1102 and the first movement space 1111 may be separated. A sensor unit carrier body 1310 of the sensor unit carrier 130 to be described later, the needle carrier 140, and the sensor unit 30 may move toward the transmission unit 40 through the first movement space 1111 in an insertion process of the transcutaneous sensor member 330 into subcutaneous tissue.

On an outer surface of one or more of the plurality of partition walls 1112, a first acceleration latch 1118 provided in a shape protruding toward the outside from the outer surface of the partition wall 1112 may be provided. The first acceleration latch 1118 may interact with a second acceleration latch 1328 provided in the sensor unit carrier 130 to be described later so as to grant a movement initiation condition of the sensor unit carrier 130 to move in the first direction only when a force of a predetermined amount or more is applied to the sensor unit carrier 130. That is, since insertion of the transcutaneous sensor member 330 into subcutaneous tissue is carried out only when a force sufficient to release a movement constraint of the second acceleration latch 1328 by the first acceleration latch 1118 is applied to the handle housing 120, a situation in which the transcutaneous sensor member 330 is arbitrarily fired in an unintended situation by a user may be effectively avoided. Meanwhile, in order for the transcutaneous sensor member 330 to be normally inserted into the subcutaneous tissue, the needle body 1402 is required to move at a speed equal to or greater than a reference speed so that a leading end of the needle body 1402 may penetrate a skin surface, and through interaction between the first acceleration latch 1118 and the second acceleration latch 1328, acceleration conditions may be granted so as to fire the needle body 1402 at a speed equal to or greater than the reference speed, thereby effectively inducing normal insertion of the transcutaneous sensor member 330 into the subcutaneous tissue. The first acceleration latch 1118 may be provided as a latch structure having a wedge-shaped cross-section provided with an inclined surface 1119a and a support surface 1119b, but a shape of the first acceleration latch 1118 is not necessarily limited thereto, and as long as the shape is capable of granting a movement initiation condition or an acceleration condition of the sensor unit carrier 130 through interaction with the second acceleration latch 1328, it may be variously modified and applied.

At a leading end portion of the partition wall 1112 provided with the first acceleration latch 1118, a bridge pressing portion 1116 having a shape in which a cross-section decreases toward a leading end may be provided. The bridge pressing portion 1116 may be provided to press a bridge 1330 provided in the sensor unit carrier 130 to be described later so as to cut the bridge 1330. That is, in an initial step of movement of the sensor unit carrier 130 in the first direction, the bridge 1330 provided in the sensor unit carrier 130 is moved to a position in close contact with the bridge pressing portion 1116, and only when a force in the first direction sufficient to cut the bridge 1330 pressed by the bridge pressing portion 1116 is applied to the sensor unit carrier 130, movement of the sensor unit carrier 130 in the first direction may be completed. When the bridge 1330 is not cut despite the movement of the sensor unit carrier 130 in the first direction, a state in which the bridge 1330 is caught by the bridge pressing portion 1116 is maintained, and the sensor unit carrier 130 may be placed in a situation where the sensor unit carrier 130 can no longer move in the first direction. That is, not only may arbitrary firing of the transcutaneous sensor member 330 in an unintended situation by a user be effectively prevented through interaction between the bridge 1330 and the bridge pressing portion 1116, but also sufficient acceleration conditions for normal insertion of the transcutaneous sensor member 330 into the subcutaneous tissue may be granted.

Between the first acceleration latch 1118 and the bridge pressing portion 1116, a carrier slit 1117 formed to penetrate in a shape incising the partition wall 1112 along a direction parallel to a direction opposite to the first direction from the leading end portion of the partition wall 1112 may be provided. In a process in which the sensor unit carrier 130 moves in a first direction, an extension arm connection portion 1322 of the sensor unit carrier 130 to be described later may be introduced into an interior of the carrier slit 1117. As the extension arm connection portion 1322 is introduced into the interior of the carrier slit 1117, interference with movement in the first direction of the extension arm connection portion 1322 by the partition wall 1112 may be excluded, and movement of the sensor unit carrier 130 may be guided by the carrier slit 1117. As the extension arm connection portion 1322 moves along the carrier slit 1117, the sensor unit 30 separably fixed to an end portion of the sensor unit carrier 130 may be accurately delivered to the seating groove 412 of the transmission unit 40.

On an inner surface of the partition wall 1112 forming the first movement space 1111, a carrier guide groove 1114 may be formed to be recessed in a direction intersecting the first direction, and the carrier guide groove 1114 may be formed to extend along the first direction. In one example, the carrier guide groove 1114 may be recessed in a shape directed from the inner surface of the partition wall 1112 toward the outside. The sensor unit carrier 130 may be provided with a carrier guide protrusion 1310a having a shape corresponding to the carrier guide groove 1114. The carrier guide protrusion 1310a may protrude from one end of the sensor unit carrier body 1310 in a direction intersecting the first direction and may be formed in a shape extending along the first direction. At least one end of the carrier guide protrusion 1310a may be disposed in the interior of the carrier guide groove 1114. A leading end portion of the carrier guide protrusion 1310a may be disposed in the carrier guide groove 1114 to guide movement of the sensor unit carrier 130 so that the sensor unit carrier 130 moves along the first direction. In the drawings, four carrier guide protrusions 1310a disposed at respective corner portions of the sensor unit carrier 130 and the carrier guide grooves 1114 formed on an inner surface of the column 1110 in positions and in a number corresponding thereto are exemplarily illustrated. However, a shape and number of the carrier guide protrusion 1310a and the carrier guide groove 1114 are not necessarily limited to matters illustrated in the drawings, and as long as the shape and number are capable of guiding movement of the sensor unit carrier 130 in the first direction, they may be variously modified and applied.

In the interior of the second interior space 1102, a partition wall support portion 1113 provided to support the column 1110 may be disposed upright. The partition wall support portion 1113 may be disposed so that one end thereof is connected to the partition wall 1112 and the other end thereof is connected to an inner side wall of the body housing body portion 1100, thereby improving structural stability and rigidity of the column 1110 and the body housing body portion 1100.

A body extension portion 1140 may be provided in a pair at one end portion of the body housing body portion 1100 in a shape extending along a direction opposite to the first direction from the one end portion of the body housing body portion 1100 where the second opening 1101 is formed. The handle housing guide groove 1142 may be formed in a shape extending along the first direction from an outer surface of a leading end portion side of a body extension portion 1140 to an outer surface of the other end portion side of the body housing body portion 1100. That is, the handle housing guide groove 1142 may be formed to be recessed so as to extend from the outer surface of the body extension portion 1140 to the outer surface of the body housing body portion 1100 in a shape terminating the body extension portion 1140 and the body housing body portion 1100. A leading end portion of the guide protrusion 1210 of the handle housing 120 may be disposed in an interior of the handle housing guide groove 1142 so that a movement direction of the handle housing 120 moving along the first direction may be guided.

On a leading end portion side of the body extension portion 1140, the second movement restriction portion 1150 may be provided. On the leading end portion of the body extension portion 1140, a movement restriction latch accommodation portion 1156 having a shape incising the body extension portion 1140 along the first direction from the leading end of the body extension portion 1140 may be formed to penetrate. A movement restriction body 1151 may be disposed to be accommodated in the movement restriction latch accommodation portion 1156, and both side end portions on a central portion side of the movement restriction body 1151 may be connected to the body extension portion 1140 by a body connection portion 1157. Since both side end portions on the central portion side of the movement restriction body 1151 are connected to the body extension portion 1140 through the body connection portion 1157, when an external force is applied to the movement restriction body 1151, the movement restriction body 1151 may be torsionally deformed in a state of being accommodated in the movement restriction latch accommodation portion 1156. On an outer surface of a one side end portion of the movement restriction body 1151, a first movement restriction latch 1152 may be provided, and on an outer surface of the other side end portion of the movement restriction body 1151, a second movement restriction latch 1154 may be provided. The first movement restriction latch 1152 and the second movement restriction latch 1154 may preferably be disposed to be spaced apart from each other along the first direction. The first movement restriction latch 1152 may be provided as a latch structure having a wedge-shaped cross-section provided with a first inclined surface 1153a and a first support surface 1153b, and likewise, the second movement restriction latch 1154 may be provided as a latch structure having a wedge-shaped cross-section provided with a second inclined surface 1155a and a second support surface 1155b. The first movement restriction latch 1152 and the second movement restriction latch 1154 may interact with the first movement restriction portion 1220 of the above-described handle housing 120 to constrain movement of the handle housing 120.

The support surface 1222 of the first movement restriction portion 1220 may be formed along a direction substantially parallel to the second direction, and the inclined surface 1221 of the first movement restriction portion 1220 may be inclinedly disposed so as to become adjacent to an inner side wall of the handle housing 120 along the first direction from an end portion of the protruding support surface 1222. The first support surface 1153b of the first movement restriction latch 1152 may be formed along a direction substantially parallel to the second direction, and the first inclined surface 1153a of the first movement restriction latch 1152 may be inclinedly disposed so as to become adjacent to one end portion side of the movement restriction body 1151 along a direction opposite to the first direction from an end portion of the protruding first support surface 1153b. The second support surface 1155b of the second movement restriction latch 1154 may be formed along a direction substantially parallel to the second direction, and the second inclined surface 1155a of the second movement restriction latch 1154 may be inclinedly disposed so as to become adjacent to the other end portion side of the movement restriction body 1151 along a direction opposite to the first direction from an end portion of the protruding second support surface 1155b.

When the handle housing 120 and the body housing 110 are assembled, in a process in which the handle housing 120 relatively moves with respect to the body housing 110 in the first direction, the inclined surface 1221 of the first movement restriction portion 1220 may reach a state of coming into contact with the first inclined surface 1153a of the first movement restriction latch 1152. Thereafter, as the handle housing 120 further moves along the first direction, one end of a movement restriction body 1151 may be torsionally deformed toward the second interior space 1102, and accordingly, the first movement restriction portion 1220 may pass through the first movement restriction latch 1152. After the first movement restriction portion 1220 passes through the first movement restriction latch 1152, the movement restriction body 1151 may be restored to a state before torsional deformation. After the first movement restriction portion 1220 passes through the first movement restriction latch 1152 and coupling of the handle housing 120 and the body housing 110 is completed, since the support surface 1222 of the first movement restriction portion 1220 and the first support surface 1153b of the first movement restriction latch 1152 are placed in a state of facing each other, even if a force in a direction opposite to the first direction is applied to the handle housing 120, the support surface 1222 of the first movement restriction portion 1220 and the first support surface 1153b of the first movement restriction latch 1152 may support each other, thereby preventing the handle housing 120 from being arbitrarily deviated from the body housing 110.

In an insertion process of the transcutaneous sensor member 330 into the subcutaneous tissue, in a state in which the inclined surface 1221 of the first movement restriction portion 1220 comes into contact with the second inclined surface 1155a of the second movement restriction latch 1154, the handle housing 120 may move in the first direction, and the other end of the movement restriction body 1151 may be torsionally deformed toward the second interior space 1102 so that the first movement restriction portion 1220 passes through the second movement restriction latch 1154. After the first movement restriction portion 1220 passes through the second movement restriction latch 1154, the movement restriction body 1151 may be restored to a state before torsional deformation. After the first movement restriction portion 1220 passes through the second movement restriction latch 1154 and the insertion of the transcutaneous sensor member 330 into the subcutaneous tissue is completed, since the support surface 1222 of the first movement restriction portion 1220 and the second support surface 1155b of the second movement restriction latch 1154 are placed in a state of facing each other, movement of the handle housing 120 in the direction opposite to the first direction may be restricted. That is, after the insertion of the transcutaneous sensor member 330 into the subcutaneous tissue, through interaction between the first movement restriction portion 1220 and the second movement restriction portion 1150, restoration of the handle housing 120 to a position before insertion of the transcutaneous sensor member 330 may be restricted, and accordingly, reuse of the applicator 10 after firing may be effectively prevented.

In one of the plurality of partition walls 1112, the fixing portion 1130 for restricting arbitrary movement of the sensor unit carrier 130 during assembly of the applicator 10 may be provided.

In a leading end portion of the partition wall 1112 provided with the fixing portion 1130, a first incision groove 1135a and a second incision groove 1135b formed to incise the partition wall 1112 along the first direction from a leading end of the partition wall 1112 may be formed to penetrate in parallel while being spaced apart from each other. Between the first incision groove 1135a and the second incision groove 1135b, a support 1134 may be disposed upright in a direction parallel to the first direction, and on one surface of the support 1134 facing the first movement space 1111, a fixing protrusion portion 1137 having a shape protruding toward the first movement space 1111 may be provided.

On the other surface of the support 1134 opposite to the one surface of the support 1134 on which the fixing protrusion portion 1137 is formed, a pressing movement extension portion 1136 extending from the other surface of the support 1134 may be provided, and on a protruding leading end portion of the pressing movement extension portion 1136, a pressing movement portion 1131 may be provided. A leading end portion of the fixing protrusion portion 1137 may be disposed in an interior of the first movement space 1111, and the fixing protrusion portion 1137 and the pressing movement extension portion 1136 may be disposed parallel along a direction substantially parallel with the second direction. The support 1134 may be disposed on a boundary between the first movement space 1111 and the second interior space 1102, and the pressing movement extension portion 1136 and the pressing movement portion 1131 may be disposed in a region corresponding to the second interior space 1102. The pressing movement portion 1131 may include a pressing movement body portion 1132 connected to the pressing movement extension portion 1136, and pressing movement wing portions 1133 provided at both side ends of the pressing movement body portion 1132. The pressing movement wing portion 1133 may be disposed spaced apart from the pressing movement extension portion 1136. The pressing movement wing portion 1133 may be provided with a pressing movement inclined surface 1133' having a shape coming close to the partition wall 1112 along the first direction. The fixing portion 1130 may be provided in a symmetric shape based on C-C' of FIG. 14.

Meanwhile, as illustrated in FIG. 17(a), a pressing movement portion 1131a may include one pressing movement wing portion 1133a. That is, a pressing movement extension portion 1136a may be provided in a shape extending from a support 1134a to a region corresponding to the second interior space 1102, and a pressing movement body portion 1132a may be provided in a shape extending from a leading end of the pressing movement extension portion 1136a toward one side. That is, the pressing movement extension portion 1136a is connected at one end thereof to the pressing movement body portion 1132a and may be disposed substantially parallel with an adjacent partition wall 1112. At the other end of the pressing movement body portion 1132a, the pressing movement wing portion 1133a is disposed, and a fixing portion 1130a may be provided in an asymmetric shape.

As illustrated in FIG. 17(b), a pressing movement inclined surface 1133b provided on a pressing movement wing portion 1133b may include a first inclined surface 1133b' and a second inclined surface 1133b" having different inclinations. The second inclined surface 1133b" is disposed relatively adjacent to the partition wall 1112 compared with the first inclined surface 1133b', and the second inclined surface 1133b" may be provided to have a relatively gentle inclination angle with respect to the second direction compared with the first inclined surface 1133b'. As the handle housing 120 moves in the first direction, the push arm 1230 moves so that its leading end portion sequentially comes into contact with the first inclined surface 1133b' and the second inclined surface 1133b", and since the second inclined surface 1133b" is provided to have a relatively gentle inclination angle with respect to the second direction compared with the first inclined surface 1133b', a boundary point may be provided for movement of the handle housing 120 in the first direction. That is, since the second inclined surface 1133b" is provided to have a relatively gentle inclination angle with respect to the second direction compared with the first inclined surface 1133b', only when the handle housing 120 is pressed in the first direction with gradually greater pressing force or is continuously pressed in the first direction, the leading end portion of the push arm 1230 may pass through the second inclined surface 1133b", and accordingly, arbitrary firing of the transcutaneous sensor member 330 in unintended situations by a user may be effectively prevented. Meanwhile, depending on use environments, there may be a case in which an initial acceleration condition of the applicator assembly 1 is important, and in this case, the pressing movement inclined surface 1133b may be provided such that the first inclined surface 1133b' has a gentle inclination angle with respect to the second direction compared with the second inclined surface 1133b". That is, since the first inclined surface 1133b' is provided to have a relatively gentle inclination angle with respect to the second direction compared with the second inclined surface 1133b", only when an initial pressure in the first direction, sufficient for the leading end portion of the push arm 1230 to pass through the first inclined surface 1133b', is applied to the handle housing 120, firing of the transcutaneous sensor member 330 may be performed, and the transcutaneous sensor member 330 may be inserted into a subcutaneous position in a state where an initial acceleration condition is granted.

As illustrated in FIG. 17(c), at one end of a pressing movement wing portion 1133c facing a transmitter accommodation portion 1104, a fixing auxiliary groove portion 1133c' formed to be recessed in a direction opposite to the first direction may be provided. In a process of movement of the handle housing 120 in the first direction, at least a portion of the pressing movement wing portion 1133c may be introduced into the wing portion accommodation hole 1231, and when the handle housing 120 attempts to move in a direction opposite to the first direction thereafter, the barrier 1231' comes into contact with and is supported by the fixing auxiliary groove portion 1133c', thereby movement of the handle housing 120 in the direction opposite to the first direction may be restricted. Since the fixing auxiliary groove portion 1133c' assists the barrier 1231' to come into contact with the pressing movement wing portion 1133c at a more accurate position, it may be preferable that the fixing auxiliary groove portion 1133c' is formed to be recessed at a position corresponding to the barrier 1231'. Meanwhile, although FIG. 17(c) illustrates a case in which the fixing auxiliary groove portion 1133c' is formed to be recessed in a region adjacent to the partition wall 1112, the fixing auxiliary groove portion 1133c' may include a case of being formed to be recessed in a central region at one end side of the pressing movement wing portion 1133c.

As illustrated in FIGS. 18(a) and 18(b), on one surface of a pressing movement body portion 1132d facing the partition wall 1112, an auxiliary inclined surface 1132d' may be provided. The auxiliary inclined surface 1132d' may be inclinedly disposed to become adjacent to the partition wall 1112 along the first direction. The auxiliary inclined surface 1132d' may be provided to have a relatively steep inclination angle with respect to the second direction compared with a pressing movement inclined surface 1133e. When the handle housing 120 moves in the first direction, the leading end portion of the push arm 1230 presses the pressing movement wing portion 1133d so that a leading end portion of a fixing protrusion portion 1137d is discharged from a fixing groove 1340, and as the handle housing 120 continuously moves in the first direction, at least one end of the pressing movement wing portion 1133d may be introduced into the wing portion accommodation hole 1231. Since the auxiliary inclined surface 1132d' is inclinedly disposed to become adjacent to the partition wall 1112 along the first direction, the fixing portion 1130 may be disposed at a position radially spaced apart from the first movement space 1111 compared with an initial position, and accordingly, it may be possible to minimize that the leading end portion of the fixing protrusion portion 1137d is reintroduced into the first movement space 1111. That is, since the auxiliary inclined surface 1132d' is provided on one surface of the pressing movement body portion 1132d facing the partition wall 1112 to become adjacent to the partition wall 1112 along the first direction, after the leading end portion of the fixing protrusion portion 1137d is discharged from the fixing groove 1340, it is possible to effectively exclude movement interference in an interior of the first movement space 1111, which may occur as the leading end portion of the fixing protrusion portion 1137d is reintroduced into the first movement space 1111.

As described below, on one surface of the sensor unit carrier body 1310, a fixing groove 1340 having a shape corresponding to the fixing protrusion portion 1137 may be formed to be recessed. As the fixing groove 1340 is formed to be recessed, a space in which at least a portion of the fixing portion 1130 is accommodated may be formed. When defining a position of the sensor unit carrier 130 in a state where the handle housing 120 is not pressed by a user (that is, a state where firing of the transcutaneous sensor member 330 and insertion into subcutaneous tissue are not performed) as an initial position, and defining a position of the sensor unit carrier 130 in a state after the handle housing 120 is pressed by the user (that is, a state after firing of the transcutaneous sensor member 330 and insertion into subcutaneous tissue are performed) as an insertion position, it may be preferable that the fixing groove 1340 is formed to be recessed on one surface of the sensor unit carrier body 1310 so that the fixing groove 1340 of the sensor unit carrier 130 positioned at the initial position and the fixing protrusion portion 1137 are positioned at a position corresponding to each other. Meanwhile, the handle housing 120 may be positioned at a first position after completion of assembly of the applicator 10, may be positioned at a second position after firing of the transcutaneous sensor member 330 is performed, and the sensor unit carrier 130 may move from an initial position to an insertion position in interlocking with the handle housing 120 as the handle housing 120 moves from the first position to the second position. In a process of fitting the sensor unit carrier body 1310 into the first movement space 1111 in order to assemble the applicator 10, the fixing protrusion portion 1137 comes into contact with one surface of the sensor unit carrier body 1310, whereby the support 1134 is torsionally deformed outward, and the fixing portion 1130 is maintained in a state of being moved in a direction away from the first movement space 1111. Subsequently, when the sensor unit carrier body 1310 is pushed into the first movement space 1111 along the first direction until the fixing protrusion portion 1137 and the fixing groove 1340 correspond to each other in position, a leading end portion of the fixing protrusion portion 1137 is introduced into and disposed in an interior of the fixing groove 1340, and the support 1134 is restored to a state before torsional deformation.

As the leading end portion of the fixing protrusion portion 1137 is introduced into the interior of the fixing groove 1340, arbitrary movement in the first direction or arbitrary movement in a direction opposite to the first direction of the sensor unit carrier 130 may be restricted. In a state where the leading end portion of the fixing protrusion portion 1137 is accommodated in the interior of the fixing groove 1340, subsequently, an assembly work of the handle housing 120 and the body housing 110 may be performed.

In a process of coupling the handle housing 120 to the body housing 110, a leading end portion of the push arm 1230 provided on the handle housing 120 may come into contact with the pressing movement inclined surface 1133' of the pressing movement wing portion 1133. As the leading end portion of the push arm 1230 moves in the first direction in a state of coming into contact with the pressing movement inclined surface 1133' and presses the pressing movement wing portion 1133, the support 1134 may be torsionally deformed outward and the pressing movement portion 1131 may be pushed in a direction away from the sensor unit carrier body 1310.

As the pressing movement portion 1131 is pushed in a direction away from the sensor unit carrier body 1310, the leading end portion of the fixing protrusion portion 1137 moves to a position deviated from the fixing groove 1340, and accordingly, the arbitrary movement restriction of the sensor unit carrier 130 by the fixing portion 1130 may be released. Since assembly of the applicator 10 may be performed in a state where the sensor unit carrier 130 maintains the initial position by the fixing portion 1130, work efficiency during the assembly work of the applicator 10 may be more effectively improved.

Meanwhile, although in the above, a case where the leading end portion of the push arm 1230 presses one end of the fixing portion 1130 in an assembly process of the applicator 10 so that the fixing protrusion portion 1137 is discharged from the fixing groove 1340 has been described as an example, it may include a case where in a firing process of the transcutaneous sensor member 330, the leading end portion of the push arm 1230 presses one end of the fixing portion 1130 so that the fixing protrusion portion 1137 is discharged from the fixing groove 1340. That is, in the assembly process of the applicator 10, the leading end portion of the push arm 1230 may be positioned at a position adjacent to one end of the fixing portion 1130 or at a position in contact with one end of the fixing portion 1130, and in a process in which a user presses the handle housing 120 so that the handle housing 120 moves from the first position to the second position, the fixing protrusion portion 1137 may be discharged from the fixing groove 1340 by the leading end portion of the push arm 1230 pressing one end of the fixing portion 1130.

On a closed one end side of the body housing 110 facing the second opening 1101, a transmission unit accommodation portion 1104 capable of accommodating the transmission unit 40 may be provided. The transmission unit accommodation portion 1104 may be formed in a shape recessed from the exterior of the body housing 110 toward the second interior space 1102. The transmission unit accommodation portion 1104 may preferably be formed to be recessed in a shape corresponding to the transmission unit housing 410 so as to be able to accommodate the transmission unit housing 410 in its interior. In a state where the transmission unit 40 is accommodated in the transmission unit accommodation portion 1104, when one end of the applicator 10 is brought into close contact with skin, the transmission unit accommodation portion 1104 may be preferably formed to be recessed to a recessed depth such that the adhesive member 430 provided in the transmission unit 40 may be attached to the skin.

At one end of the body housing 110 where the transmission unit accommodation portion 1104 is formed, a third opening 1106 connecting the transmission unit accommodation portion 1104 and the first movement space 1111 may be formed to penetrate. In an insertion process of the transcutaneous sensor member 330 into the subcutaneous tissue, the sensor unit 30 that has moved along the first direction from the first movement space 1111 may pass through the third opening 1106 and be delivered to the seating groove 412 of the transmission unit 40 accommodated in the transmission unit accommodation portion 1104.

At both side ends of the transmission unit accommodation portion 1104, transmission unit support portions 1160 for preventing arbitrary deviation of the transmission unit 40 from the transmission unit accommodation portion 1104 may be provided. At both side ends of the transmission unit accommodation portion 1104, support hook accommodation spaces 1161 formed to penetrate a closed one end of the body housing 110 may be provided. A transmission unit support hook 1162 provided to be capable of supporting the transmission unit 40 may be disposed to be accommodated in the support hook accommodation space 1161. The transmission unit support hook 1162 may be supported by support hook connection portions 1166, which extend respectively from an end portion of the body housing 110 forming the support hook accommodation space 1161 and are connected to both side ends of the transmission unit support hook 1162. Since the transmission unit support hook 1162 is connected to the support hook connection portion 1166 extending from the end portion of the body housing 110 and disposed in the support hook accommodation space 1161, when an external force is applied to the transmission unit support hook 1162, the support hook connection portion 1166 may be torsionally deformed so that the position of the transmission unit support hook 1162 may be changed.

The transmission unit support hook 1162 may include a catching portion 1164 formed to protrude in a direction toward the transmission unit accommodation portion 1104 and a pushed portion 1165 formed to protrude in a direction opposite to the transmission unit accommodation portion 1104. A leading end portion of the catching portion 1164 may be inserted into the transmission unit housing groove 417 of the transmission unit 40 accommodated in the transmission unit accommodation portion 1104, and the transmission unit 40 accommodated in the transmission unit accommodation portion 1104 may be supported by the catching portion 1164 having its leading end portion inserted into the transmission unit housing groove 417, thereby preventing arbitrary deviation from the transmission unit accommodation portion 1104. The pushed portion 1165 may be formed with a pushed portion inclined surface 1165a inclinedly disposed in a shape becoming closer to the transmission unit accommodation portion 1104 along the first direction. By pressing the pushed portion inclined surface 1165a with an extension arm pushing portion 1326 of the sensor unit carrier 130 to be described later, the transmission unit support hook 1162 may be torsionally moved in a direction away from the transmission unit accommodation portion 1104. That is, in the insertion process of the transcutaneous sensor member 330 into the subcutaneous tissue, the sensor unit carrier 130 moves together with the sensor unit 30 along the first direction, and at the same time when the sensor unit 30 is delivered from the sensor unit carrier 130 to the transmission unit 40, or at a point in time somewhat earlier than a point in time when the sensor unit 30 is delivered from the sensor unit carrier 130 to the transmission unit 40, the extension arm pushing portion 1326 may press the pushed portion inclined surface 1165a so that movement restriction of the transmission unit 40 by the catching portion 1164 may be released. Meanwhile, in a process of disposing the transmission unit 40 in the transmission unit accommodation portion 1104 in manufacturing of the applicator assembly 1, the transmission unit support hook 1162 may be pressed by the transmission unit housing 410 to be torsionally moved in a direction away from the transmission unit accommodation portion 1104, and when the transmission unit 40 is fully seated in the transmission unit accommodation portion 1104, the torsionally moved transmission unit support hook 1162 may be restored to its original state so that a leading end portion of the catching portion 1164 may be disposed in an interior of the transmission unit housing groove 417. The transmission unit support portion 1160 is provided so as to release movement constraint of the transmission unit 40 only when intended by a worker or a user in an assembly process or a using process of the applicator assembly 1, and thus, assembling work convenience and convenience of use of the applicator assembly 1 may be more effectively improved.

On one or more inner surfaces of the partition wall 1112 forming the first movement space 1111, a grip arm guide groove 1120 extending along the first direction may be formed in a shape recessed toward the outside. A grip arm 1422 of the needle carrier 140 to be described later may be disposed in the grip arm guide groove 1120 so that a movement direction of the needle carrier 140 may be guided. At a central portion of the grip arm guide groove 1120, a step portion 1121 protruding to a height substantially corresponding to one surface of the partition wall 1112 in which the grip arm guide groove 1120 is formed may be formed, and at one end and the other end of the step portion 1121, a first step inclined surface 1123 and a second step inclined surface 1124 may respectively be formed. The first step inclined surface 1123 may be inclinedly disposed in a shape becoming adjacent to the first movement space 1111 along the first direction, and the second step inclined surface 1124 may be inclinedly disposed in a shape away from the first movement space 1111 along the first direction.

During assembly of the applicator assembly 1, the needle carrier 140 may be introduced into an interior of the first movement space 1111 along the first direction, and in a state where the grip arm 1422 is disposed in the grip arm guide groove 1120, the needle carrier 140 may move to an initial position. When the needle carrier 140 is disposed in the initial position, a grip arm protrusion portion 1424 formed on the grip arm 1422 may pass through the first step inclined surface 1123 or be positioned at a position immediately before passing through the first step inclined surface 1123. Subsequently, as the needle carrier 140 further moves along the first direction, the grip arm protrusion portion 1424 may come into close contact with the step portion 1121 and pressed inward, and in a process in which the needle carrier 140 moves, the sensor unit 30 may be maintained in a more firmly fixed state on the inwardly pressed grip arm 1422. Meanwhile, in a process in which the needle carrier 140 further moves along the first direction to move to an insertion position, the grip arm protrusion portion 1424 may pass through the second step inclined surface 1124, and as the grip arm protrusion portion 1424 passes through the second step inclined surface 1124, pressing of the grip arm protrusion portion 1424 by the step portion 1121 may be released. That is, in the insertion process of the transcutaneous sensor member 330 into the subcutaneous tissue, the pressing of the grip arm protrusion portion 1424 by the step portion 1121 may be released simultaneously with delivering the sensor unit 30 to the seating groove 412 of the transmission unit 40 or before delivering the sensor unit 30 to the seating groove 412 of the transmission unit 40. It is possible not only to effectively prevent the sensor unit 30 from being deviated from a designated position in an interior of the applicator 10 during the insertion process of the transcutaneous sensor member 330 into the subcutaneous tissue, but also to effectively prevent a phenomenon in which the sensor unit 30 is brought along in a direction opposite to the first direction by the grip arm 1422 immediately after the insertion of the transcutaneous sensor member 330 into the subcutaneous tissue.

### Sensor unit carrier

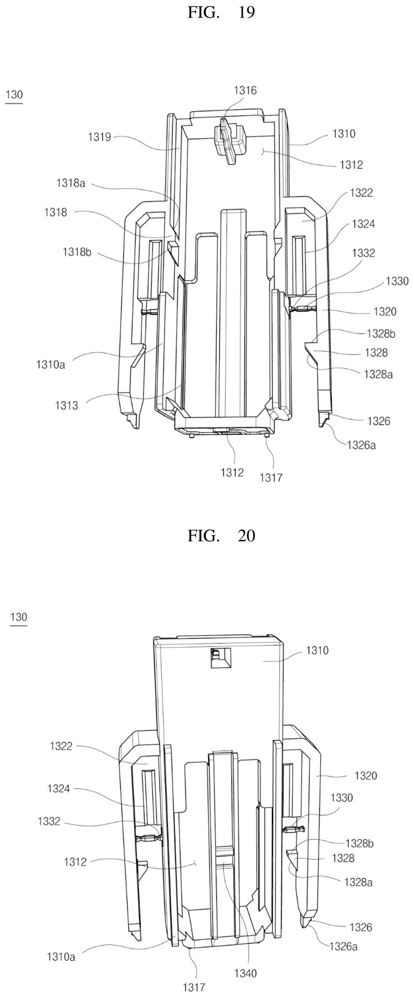

FIG. 19 and FIG. 20 are perspective views illustrating an exemplary embodiment of the sensor unit carrier 130.

The sensor unit carrier 130 may be disposed in an interior of the applicator assembly 1 so as to move together with the needle carrier 140 and the sensor unit 30 in the first direction in the insertion process of the transcutaneous sensor member 330 into the subcutaneous tissue. After the insertion of the transcutaneous sensor member 330 into the subcutaneous tissue, the needle carrier 140 may move to a retracted position such that the needle body 1402 is discharged from the skin, whereas the sensor unit carrier 130 may be maintained at an insertion position.

The sensor unit carrier 130 includes the sensor unit carrier body 1310 constituting a skeleton of the sensor unit carrier 130, and the sensor unit carrier body 1310 may be provided with a second movement space 1312 having a front side opened. The needle carrier 140 may move to the retracted position through the second movement space 1312 after the insertion of the transcutaneous sensor member 330 into the subcutaneous tissue. At one end portion of the sensor unit carrier body 1310 adjacent to the third opening 1106 formed in the body housing 110, a fourth opening 1312 connecting the exterior and the second movement space 1312 may be formed to penetrate. The needle body 1402 may protrude to the outside of the sensor unit carrier body 1310 through the fourth opening 1312. On one end surface of the sensor unit carrier body 1310 facing the third opening 1106, a fixing protrusion 1317 having a shape protruding toward the third opening 1106 may be provided, and the sensor unit 30 may be more firmly fixed to the sensor unit carrier 130 by a leading end portion of the fixing protrusion 1317 inserted into an interior of the fixing groove 317 of the sensor unit 30.

At a corner portion on a side end side of the sensor unit carrier body 1310, the carrier guide protrusion 1310a having a shape protruding toward the outside may be formed to extend along the first direction. A leading end portion of the carrier guide protrusion 1310a may be disposed in an interior of the carrier guide groove 1114 formed in the column 1110, and a movement direction of the sensor unit carrier body1310 moving along the first direction may be guided by the carrier guide protrusion 1310a and the carrier guide groove 1114. On an inner side surface of the sensor unit carrier body 1310 forming the second movement space 1312, a needle carrier guide protrusion 1313 formed to protrude from the inner side surface of the sensor unit carrier body 1310 toward a central portion of the second movement space 1312 and to extend along the first direction may be provided. A leading end portion of the needle carrier guide protrusion 1313 may be disposed in an interior of a needle guide groove 1415 formed in the needle carrier body 1410 to be described later, and a movement direction of the needle carrier 140 moving in a direction opposite to the first direction may be guided by the needle carrier guide protrusion 1313 and the needle guide groove 1415.

On an inner side surface of the sensor unit carrier body 1310 adjacent to the handle housing 120, a sliding groove 1319 formed to be recessed from an inner surface on a leading end side of the sensor unit carrier body 1310 and to extend along the first direction may be provided. It is preferable that the sliding groove 1319 is formed at a position adjacent to an opened front surface side of the second movement space 1312. A sensor unit carrier detent 1318 having a detent inclined surface 1318a and a detent constraint surface 1318b formed therein may be disposed in the sliding groove 1319. Since a needle carrier latch 1434 of the needle carrier 140 to be described later maintains a state of being constrained to the sensor unit carrier detent 1318, the needle carrier 140 may move together with the sensor unit carrier 130 in the first direction in the insertion process of a transcutaneous sensor member 330 into the subcutaneous tissue. The detent constraint surface 1318b may be formed along a direction substantially parallel to the second direction, and the detent inclined surface 1318a may be inclinedly disposed so as to be adjacent to the sliding groove 1319 along a direction opposite to the first direction from a protruding one end of the detent constraint surface 1318b.

An extension arm 1320 may be provided in a form of protruding toward the exterior from both outer surfaces of the sensor unit carrier body 1310 and extending along the first direction. An extension arm connection portion 1322 may be provided between the sensor unit carrier body 1310 and the extension arm 1320. In the extension arm connection portion 1322, an extension arm guide groove 1324 may be formed to be recessed along the first direction. It may be preferable that the extension arm guide groove 1324 is formed in a position and shape corresponding to the carrier slit 1117 of the column 1110. In a process in which the sensor unit carrier body 1310 moves along the first direction to insert the transcutaneous sensor member 330 into the subcutaneous tissue, the extension arm connection portion 1322 having the extension arm guide groove 1324 formed therein may be introduced into the carrier slit 1117 of the column 1110, and not only a movement direction of the sensor unit carrier 130 moving in the first direction may be guided by the carrier slit 1117 and the extension arm guide groove 1324, but also interference with movement of the sensor unit carrier 130 in the first direction by the column 1110 may be excluded.

On one surface of the extension arm 1320 facing the sensor unit carrier body 1310, the second acceleration latch 1328 formed to protrude toward the sensor unit carrier body 1310 may be provided. The second acceleration latch 1328 may include a support surface 1328b formed along a direction substantially parallel to the second direction, and an inclined surface 1328a inclinedly disposed so as to be gradually adjacent toward the one surface of the extension arm 1320 along the first direction from a protruding end portion of the support surface 1328b. The second acceleration latch 1328 may prevent arbitrary firing of the applicator 10 in an unintended situation by a user through interaction with the first acceleration latch 1118 provided in the body housing 110, may grant sufficient acceleration conditions to the needle body 1402 during the insertion of the transcutaneous sensor member 330 into the subcutaneous tissue, and may prevent the sensor unit carrier 130 from moving in a direction opposite to the first direction after the insertion of the transcutaneous sensor member 330 into the subcutaneous tissue.

When the sensor unit carrier 130 is positioned at an initial position, the inclined surface 1119a of the first acceleration latch 1118 and the inclined surface 1328a of the second acceleration latch 1328 may be maintained in a state spaced apart from each other while facing each other, or in a state in contact with each other. When a user presses the handle housing 120 in the first direction such that the sensor unit carrier 130 moves in the first direction, the inclined surface 1119a of the first acceleration latch 1118 and the inclined surface 1328a of the second acceleration latch 1328 may move while rubbing against each other in a state of being in contact, and in this case, the extension arm 1320 may be torsionally deformed toward the outward direction. Subsequently, as the sensor unit carrier 130 moves toward the first direction, contact between the inclined surface 1328a of the second acceleration latch 1328 and the inclined surface 1119a of the first acceleration latch 1118 may be released, and the support surface 1119b of the first acceleration latch 1118 and the support surface 1328b of the second acceleration latch 1328 may be switched to a state facing each other. After the support surface 1119b of the first acceleration latch 1118 and the support surface 1328b of the second acceleration latch 1328 are switched to a state facing each other, the insertion of the transcutaneous sensor member 330 into the subcutaneous tissue may be performed, and after the insertion of the transcutaneous sensor member 330 into the subcutaneous tissue, movement of the sensor unit carrier 130 in a direction opposite to the first direction may be restricted by the facing support surface 1119b of the first acceleration latch 1118 and the support surface 1328b of the second acceleration latch 1328. In the above, although a latch structure having a wedge-shaped cross-section provided with the inclined surfaces 1119a and 1328a and the support surfaces 1119b and 1328b has been exemplarily described in relation to the shape of the first acceleration latch 1118 and the second acceleration latch 1328, the shape of the first acceleration latch 1118 and the second acceleration latch 1328 is not necessarily limited thereto, and may be variously modified and applied as long as the structure can prevent arbitrary firing of the applicator 10 in an unintended situation by a user through interaction, grant sufficient acceleration conditions to the needle body 1402 during the insertion of the transcutaneous sensor member 330 into the subcutaneous tissue, and prevent movement of the sensor unit carrier 130 in the direction opposite to the first direction after the insertion of the transcutaneous sensor member 330 into the subcutaneous tissue.

The bridge 1330 may be disposed between the extension arm 1320 and the sensor unit carrier body 1310. One end and the other end of the bridge 1330 may be provided to be connected to one side surface of the sensor unit carrier body 1310 and to one surface of the extension arm 1320 facing the sensor unit carrier body 1310, respectively. It may be preferable that the bridge 1330 is formed at a position corresponding to the bridge pressing portion 1116 of the column 1110, and one or more vulnerable portions 1332 having a relatively small thickness may be formed in the bridge 1330. In a process in which a user presses the handle housing 120 in the first direction so that the sensor unit carrier 130 moves in the first direction, only when the bridge 1330, which comes into contact with the bridge pressing portion 1116, is cut, the sensor unit carrier 130 may further move to an insertion position. The vulnerable portion 1332 is a region intended to be cut when the bridge 1330 is pressed by the bridge pressing portion 1116, and it is preferable that the vulnerable portion 1332 is designed to be cut only when a force applied as a user presses the handle housing 120 to insert the transcutaneous sensor member 330 into the subcutaneous tissue is applied to the bridge 1330. Here, typically, the force applied as a user presses the handle housing 120 to insert the transcutaneous sensor member 330 into the subcutaneous tissue does not generally mean a force applied in an assembling and transporting process of the applicator assembly 1, but may mean a level of force applied to the handle housing 120 by a user of the applicator assembly 1 intending insertion of the transcutaneous sensor member 330 into the subcutaneous tissue. Meanwhile, although a region having a thickness smaller than a thickness of the bridge 1330 has been described as one example of the vulnerable portion 1332, the vulnerable portion 1332 is not necessarily limited to a structure having a relatively small thickness. The vulnerable portion 1332 may mean a region having lower fracture strength than the entire bridge 1330 by applying different materials or the like, or by applying a vulnerable structure. Through interaction between the bridge 1330 and the bridge pressing portion 1116, not only arbitrary firing of the applicator 10 in unintended situations by a user may be prevented, but also sufficient acceleration conditions may be granted to the needle body 1402 during insertion of the transcutaneous sensor member 330 into the subcutaneous tissue. Meanwhile, although, in the above, a case where the bridge 1330 is provided on the sensor unit carrier 130 and the bridge pressing portion 1116 is provided on a leading end portion of the partition wall 1112 has been exemplarily described, it may include a case where a bridge structure is provided on the partition wall 1112 and a pressing portion for cutting the bridge structure is provided on the sensor unit carrier 130.

At an end portion of the extension arm 1320, the extension arm pushing portion 1326 having an inclined surface 1326a may be formed to protrude toward the first direction. When the sensor unit carrier 130 moves along the first direction and is positioned at an insertion position, the extension arm pushing portion 1326 may press the pushed portion 1165 provided in the transmission unit support hook 1162, and by the pushed portion 1165 being pressed by the extension arm pushing portion 1326, the transmission unit support hook 1162 may be torsionally deformed toward the outside away from the transmission unit 40, thereby releasing movement restriction of the transmission unit 40 by the catching portion 1164. The inclined surface 1326a formed in the extension arm pushing portion 1326 and the inclined surface 1165a formed in the pushed portion 1165 are not particularly limited as long as the shape allows the transmission unit support hook 1162 to be torsionally deformable toward the outside away from the transmission unit 40 when the pushed portion 1165 is pressed by the extension arm pushing portion 1326. It may be preferable that the extension arm pushing portion 1326 is formed at a position capable of pressing the pushed portion 1165 simultaneously with the sensor unit carrier 130 reaching the insertion position or immediately before the sensor unit carrier 130 reaches the insertion position.

At one end portion of the second movement space 1312 adjacent to the handle housing 120, a sensor unit carrier ring portion 1316 to which an elastic member 150 to be described later is fixedly disposed may be provided. Meanwhile, on one surface of the sensor unit carrier body 1310 opposite the second movement space 1312, a fixing groove 1340 having a shape corresponding to the fixing protrusion portion 1137 may be formed to be recessed, and it may be preferable that the fixing groove 1340 is formed to be recessed at a position corresponding to the fixing protrusion portion 1137 when the sensor unit carrier 130 is positioned at an initial position.

### Needle carrier

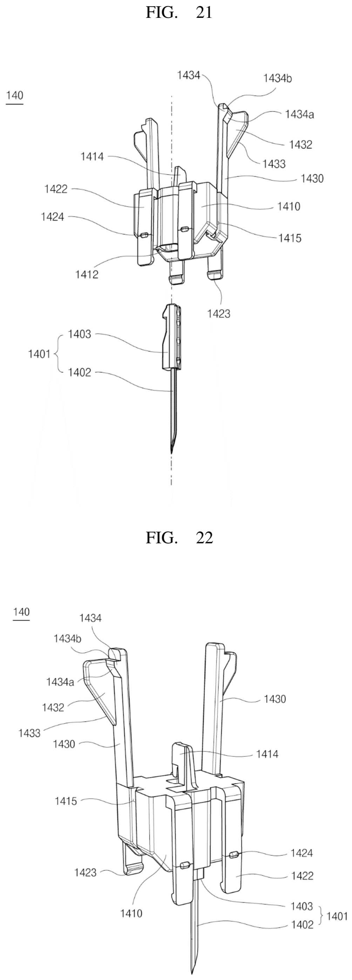

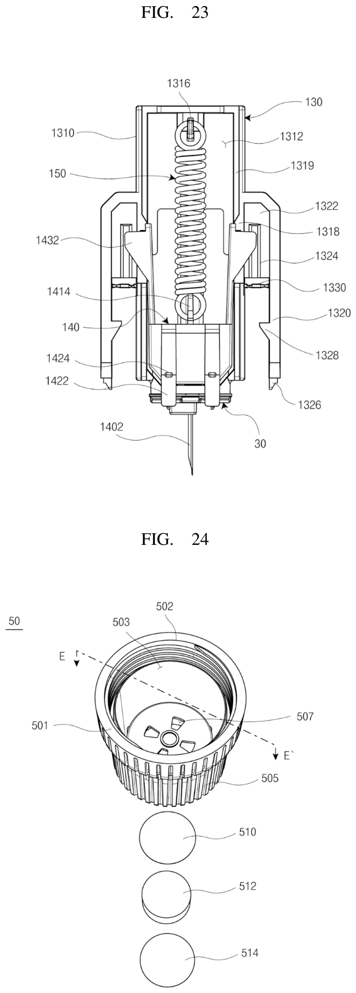

FIG. 21 is a perspective view exemplarily illustrating a coupling relationship of the needle carrier 140, FIG. 22 is a perspective view illustrating an exemplary embodiment of the needle carrier 140, and FIG. 23 is a front view exemplarily illustrating a coupling relationship between the needle carrier 140 and the sensor unit carrier 130.

The needle carrier 140 may move along the first direction together with the sensor unit carrier 130 and the sensor unit 30 by being provided with the needle 1401 for the insertion of the transcutaneous sensor member 330 into the subcutaneous tissue, and may be provided to move in a direction opposite to the first direction in the second movement space 1312 after the insertion of the transcutaneous sensor member 330 into the subcutaneous tissue to remove the needle body 1402 from the subcutaneous tissue.

The needle carrier 140 may include the needle 1401 and the needle carrier body 1410. The needle carrier body 1410 may fix the needle 1401 and be provided to move together with the needle 1401. The needle 1401 may include the needle body 1402 provided such that one end thereof is inserted into the subcutaneous tissue for the insertion of the transcutaneous sensor member 330 into the subcutaneous tissue, and a needle holder 1403 for fixing the needle body 1402. The needle body 1402 may have an opening portion opened radially outward based on an extension direction of the needle body 1402, and the opening portion may be formed to extend along the first direction. The needle body 1402 may have one side end portion adjacent to the transmission unit accommodation portion 1104 opened. One side end portion of the needle body 1402 adjacent to the transmission unit accommodation portion 1104 may be formed to be inclined. At one end of the needle carrier body 1410 facing the fourth opening 1312, a needle holder insertion groove 1412 into which the needle holder 1403 is inserted and fixed may be formed to be recessed. By the needle holder 1403 being inserted and fixed in the needle holder insertion groove 1412, the needle 1401 may be fixed to the needle carrier body 1410. When the needle carrier 140 is positioned at an initial position and an insertion position, the needle body 1402 may be discharged to the exterior through the fourth opening 1312, and when the needle carrier 140 is restored to a retracted position, the needle body 1402 may be introduced through the fourth opening 1312 and disposed in the interior of the second movement space 1312.

On one side surface of the needle carrier body 1410, the needle guide groove 1415 having a shape recessed from one side surface of the needle carrier body 1410 and extending along the first direction may be provided. A leading end portion of the above-described needle carrier guide protrusion 1313 may be disposed in the interior of the needle guide groove 1415, and by interaction between the needle carrier guide protrusion 1313 and the needle guide groove 1415, the movement of the needle carrier 140 in which the needle carrier 140 moves in a direction opposite to the first direction may be guided.

A needle carrier wing body 1430 may be provided in a pair in a shape extending toward a direction opposite to the first direction from both side ends of the needle carrier body 1410. The needle carrier wing body 1430 may be torsionally deformed upon application of an external force and may be provided to be restored to an original state after removal of the external force. At a leading end portion of the needle carrier wing body 1430, a needle carrier latch 1434 and a trigger 1432 may be provided. The needle carrier latch 1434 may include a constraint surface 1434b formed in a direction substantially parallel to the second direction, and an inclined surface 1434a inclinedly disposed to become closer to the needle carrier wing body 1430 toward the first direction from one end of the protruded constraint surface 1434b. The trigger 1432 may be provided to protrude outward relative to the needle carrier latch 1434. The trigger 1432 may be provided with a trigger inclined portion 1433 so as to have a shape in which a cross-section of the trigger 1432 decreases along the first direction. The trigger 1432 may preferably be disposed at the outside of the second movement space 1312.

Before operation of the applicator assembly 1 or when the needle carrier 140 moves along the first direction, since the constraint surface 1434b of the needle carrier latch 1434 maintains a state of being in contact with the detent constraint surface 1318b of the sensor unit carrier detent 1318, relative movement of the needle carrier 140 with respect to the sensor unit carrier 130 may be restricted. That is, in a state where the constraint surface 1434b of the needle carrier latch 1434 is in contact with the detent constraint surface 1318b of the sensor unit carrier detent 1318, independent movement of the needle carrier 140 is impossible, and the needle carrier 140 may move together only when the sensor unit carrier 130 moves in a state in which the needle carrier 140 is constrained to the sensor unit carrier 130. When the needle carrier 140 moves to a position adjacent to the insertion position in the insertion process of the transcutaneous sensor member 330 into the subcutaneous tissue, the trigger inclined portion 1433 of the trigger 1432 comes into contact with a leading end portion of the partition wall 1112, so that the needle carrier wing body 1430 may be torsionally deformed toward the inside of the second movement space 1312. As the needle carrier wing body 1430 is torsionally deformed toward the inside of the second movement space 1312, the contact constraint of the constraint surface 1434b of the needle carrier latch 1434 with the detent constraint surface 1318b of the sensor unit carrier detent 1318 may be released. By releasing the contact constraint of the constraint surface of the needle carrier latch 1434 from the detent constraint surface 1318b of the sensor unit carrier detent 1318, the needle carrier latch 1434 may ride over the sensor unit carrier detent 1318, and by an elastic force applied from the elastic member 150, the needle carrier 140 may move in a direction opposite to the first direction and reach a retracted position. To achieve purposes such as preventing pain due to excessive insertion of the human body B of the needle body 1402, and the like, it may be preferable that movement constraint of the sensor unit carrier 130 with respect to the needle carrier 140 be released before the sensor unit carrier 130 reaches the insertion position. In this case, even if the needle carrier 140 moves in the direction opposite to the first direction, the sensor unit carrier 130 maintains a state of pressing the sensor unit 30 toward the first direction, so that the transcutaneous sensor member 330 may be accurately inserted into an intended subcutaneous position by the inherent rigidity of the transcutaneous sensor member 330.

A plurality of grip arms 1422 may be provided in a shape extending along the first direction from other both side ends of the needle carrier body 1410. At a leading end portion of the grip arm 1422, a grip portion 1423 having a shape protruding inward may be provided, and a side end portion of the sensor unit housing 310 may be gripped by the grip portion 1423. The grip arm protrusion portion 1424 may be formed to protrude from one surface of the grip arm 1422 facing the partition wall of the column 1110, and when the needle carrier 140 is positioned at an initial position, the grip arm protrusion portion 1424 may be disposed in an interior of the grip arm guide groove 1120 before passing through the first step inclined surface 1123 or may maintain a state of being in close contact and pressed by the step portion 1121 after passing through the first step inclined surface 1123. In the insertion process of the transcutaneous sensor member 330 into the subcutaneous tissue, the grip arm protrusion portion 1424 may pass through the second step inclined surface 1124, and the close-contact pressing of the grip arm protrusion portion 1424 by the step portion 1121 may be released, and in a state where each of the grip arms 1422 is somewhat spread outward, the sensor unit 30 may be delivered to the transmission unit 40.

At one end of the needle carrier body 1410 facing the sensor unit carrier ring portion 1316, a needle carrier ring portion 1414 may be provided, and the other end portion of the elastic member 150, of which one end is connected to the sensor unit carrier ring portion 1316, may be connected to the needle carrier ring portion 1414. The elastic member 150 may be applied without limitation as long as it is a means capable of providing a driving force when the needle carrier 140 moves in a direction opposite to the first direction, but a tension spring may be preferably applied.

### Cap

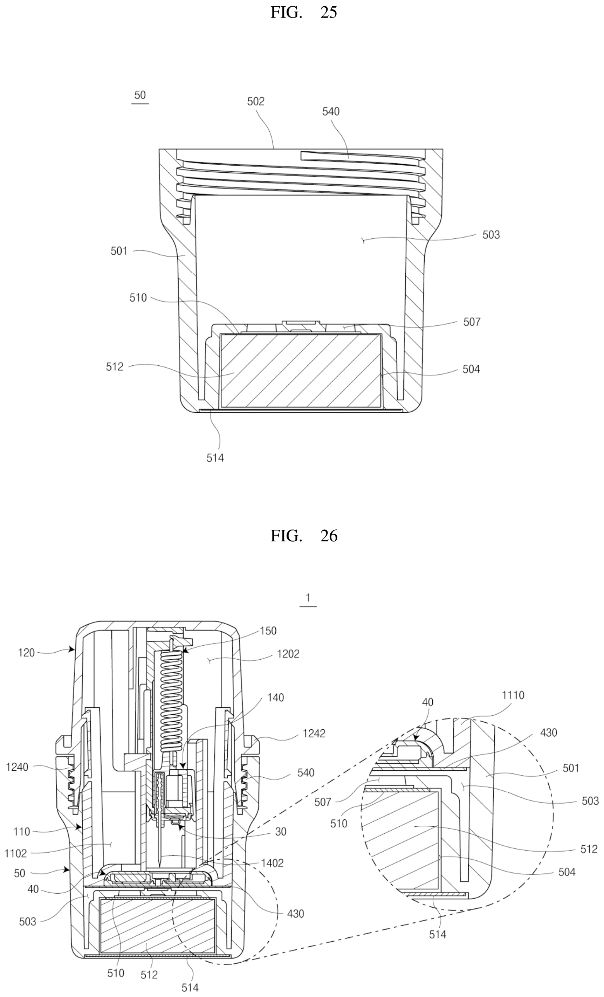

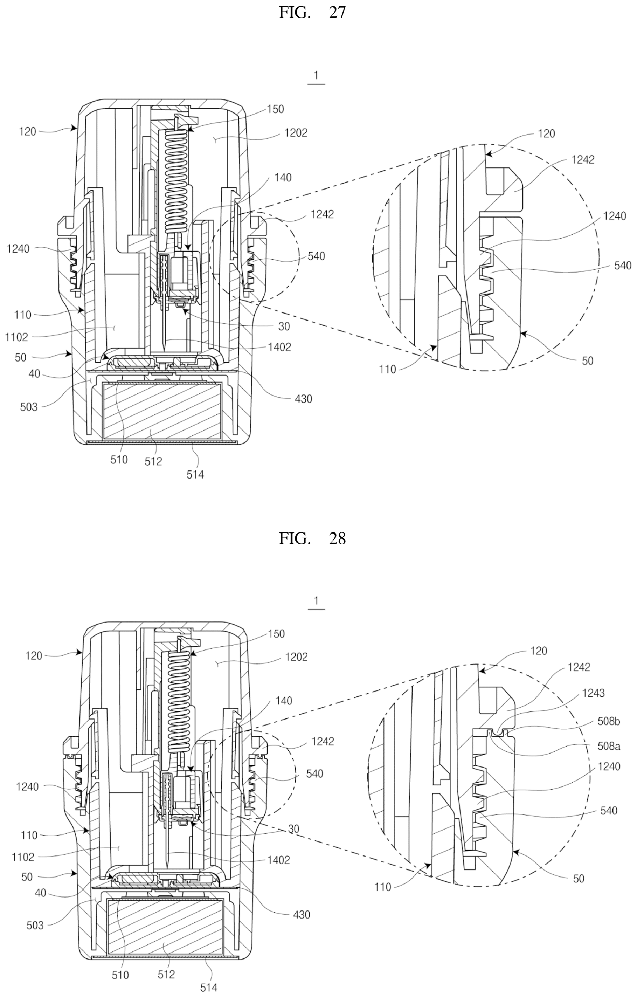

FIG. 24 is an exploded perspective view illustrating an exemplary embodiment of the cap 50, FIG. 25 is a cross-sectional view of the cap 50 cut along the E-E' direction in FIG. 24. FIGS. 26 to 28 are partially enlarged cross-sectional views illustrating an exemplary embodiment of the applicator assembly 1 to which the cap 50 is applied.

The cap 50 may be fixedly disposed so as to be releasable at one end of the applicator 10. Since the applicator assembly 1 includes the cap 50, it may effectively prevent unintended arbitrary firing of the applicator 10 or introduction of external contaminants or moisture into the interior of the applicator assembly 1, which may occur in manufacturing, distribution, storage, and use processes of the applicator assembly 1.

The cap 50 may include a cap housing 501 forming an external shape of the cap 50. In an interior of the cap housing 501, an accommodation space 503 communicating with the exterior through a fifth opening 502 formed at one end of the cap housing 501 may be formed. On an inner surface at one end of the cap housing 501 where the fifth opening 502 is formed, the screw threaded portion 540 having a shape corresponding to the screw threaded portion 1240 formed on the handle housing 120 may be provided. As the screw threaded portion 1240 formed on the handle housing 120 is screw-coupled to the screw threaded portion 540 formed on the cap housing 501, the cap 50 may be separably screw-coupled to one end of the handle housing 120. On an outer surface of the cap housing 501, a plurality of grip grooves 505 may be formed to be recessed in order to facilitate the work by a worker or user or the convenience of use. Meanwhile, although a cup-shaped cap housing 501 is illustrated in the drawings, the shape of the cap housing 501 of the present invention is not necessarily limited thereto, and the shape of the cap housing 501 may be variously modified and applied as long as it can protect and dehumidify the interior of the applicator 10. However, since one end of the cap housing 501 in which the screw threaded portion 540 is formed is intended to be screw-coupled to the screw threaded portion 1240 formed on the handle housing 120, it may be preferable that the end portion of the cap housing 501 in which the screw threaded portion 540 is formed be provided in a cylindrical shape corresponding to the end portion of the handle housing 120.

At the other closed end portion of the cap housing 501 facing the fifth opening 502, an accommodation groove 504 having a shape recessed from the outside toward the accommodation space 503 may be provided. At the other end portion of the cap housing 501 forming the accommodation groove 504, a plurality of ventilation holes 507 communicating the accommodation groove 504 with the accommodation space 503 may be formed to penetrate. In the accommodation groove 504, a first sealing member 510, a dehumidifier 512, and a second sealing member 514 may be sequentially disposed from one side of the ventilation holes 507 toward the outside. The first sealing member 510 may preferably be manufactured using a material that allows air to pass through but does not allow moisture to pass through, and as a non-limiting example, Tyvek material of DuPont may be used. By means of the first sealing member 510, introduction of moisture into the accommodation space 503 may be effectively prevented. The dehumidifier 512 may preferably be manufactured using a material having a dehumidifying function applied to an electronic device or a medical device. By means of the dehumidifier 512, moisture introduced into the accommodation space 503 may be removed. The second sealing member 514 may be disposed so as to seal the accommodation groove 504 from the exterior, and may be manufactured by applying a material that prevents the passage of moisture and external contamination sources. As a non-limiting example, the second sealing member 514 may be manufactured using an aluminum packaging sheet. Since the first sealing member 510, the dehumidifier 512, and the second sealing member 514 are sequentially disposed in the accommodation groove 504 communicating with the accommodation space 503 through the ventilation holes 507, not only can moisture introduced into the interior of the accommodation space 503 be effectively removed, but also introduction of contaminants or moisture from the exterior into the interior of the accommodation space 503 can be effectively prevented.

As illustrated in FIG. 26, sealing members 508a and 508b, which are disposed upright in a ring shape, may be provided in a pair at a leading end portion of the cap housing 501, and a calking member 1243 may be protrudingly formed on one surface of the locking protrusion portion 1242 corresponding to the leading end portion of the cap housing 501. When the screw threaded portion 540 formed in the cap housing 501 and the screw threaded portion 1240 formed in the handle housing 120 are screw-coupled to each other such that the cap 50 is coupled to one end of the handle housing 120, the sealing members 508a and 508b may closely contact and press the calking member 1243 to effectively prevent introduction of external contaminants or moisture into the space between the handle housing 120 and the cap 50. The calking member 1243 and the sealing members 508a and 508b may include a case where they are integrally injection-molded together with the handle housing 120 and the cap housing 501, or a case where they are manufactured using a material having inherent elasticity such as rubber or silicone.

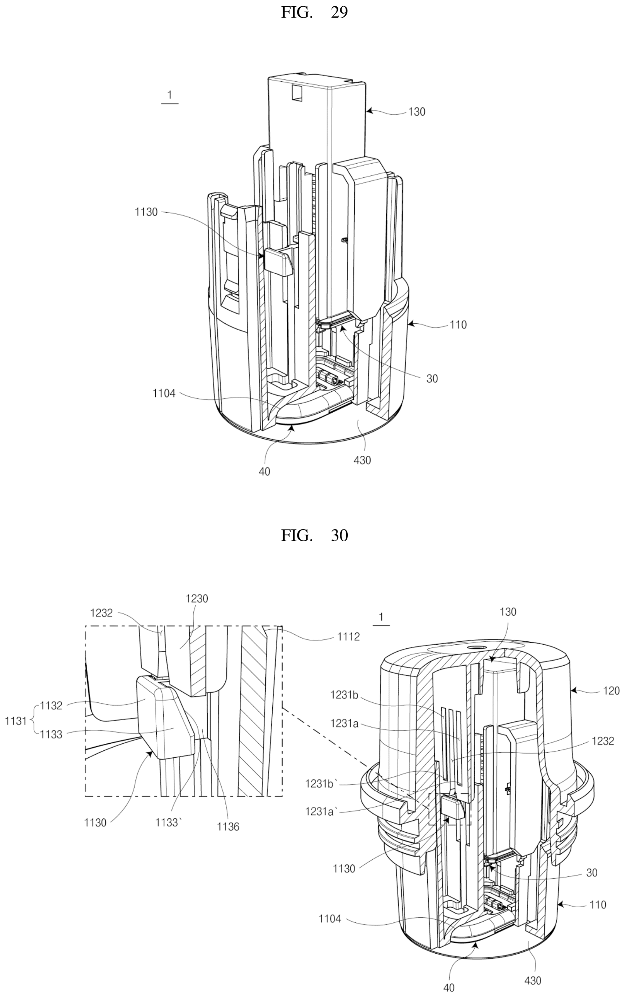

Hereinafter, with reference to FIGS. 29 to 48, the assembly and operation processes of the applicator 10 and the applicator assembly 1 will be described in more detail.

### Exemplary embodiment of action of fixing portion

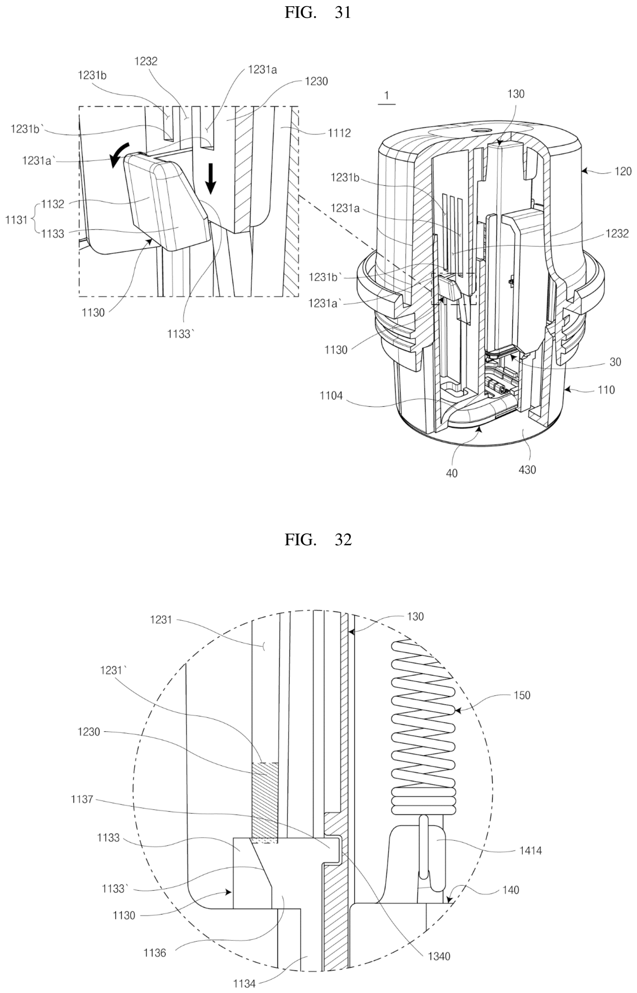

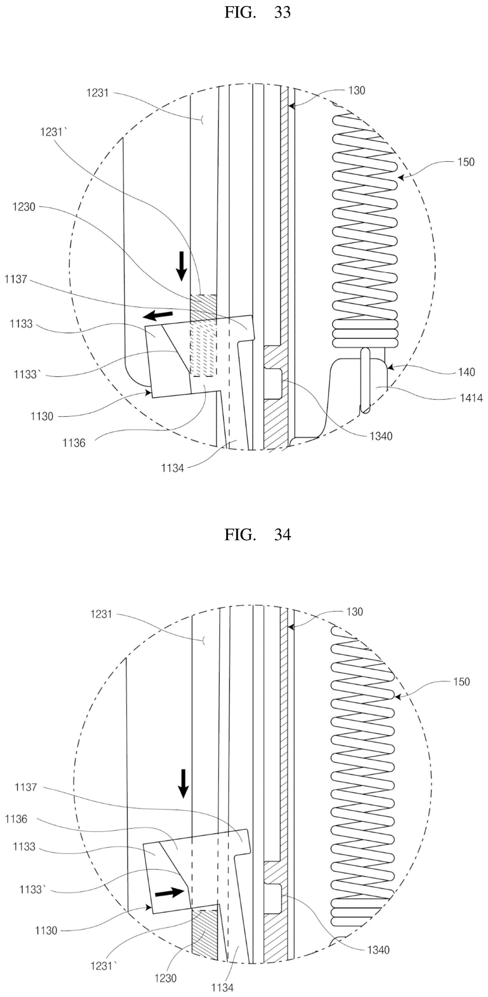

FIG. 29 is a partial cross-sectional view illustrating an exemplary embodiment in a state where the sensor unit carrier 130 is coupled to the body housing 110 during an assembly process of the applicator assembly 1, and FIGS. 30 and 31 are partial cross-sectional views illustrating an exemplary embodiment of a process of coupling the handle housing 120 to the body housing 110 to which the sensor unit carrier 130 is coupled during the assembly process of the applicator assembly 1. FIG. 32 is an enlarged cross-sectional view illustrating an exemplary embodiment of a state in which a leading end portion of the fixing protrusion portion 1137 is inserted into the fixing groove 1340 during an assembly process of the applicator assembly 1, and FIGS. 33 to 35 are enlarged cross-sectional views illustrating exemplary embodiments of interaction between the push arm 1230 and the fixing portion 1130 during assembling and operation processes of the applicator assembly 1.

As illustrated in FIGS. 29 and 32, in a process of assembling the body housing 110 and the sensor unit carrier 130, the sensor unit carrier body 1310 may be fitted into the column 1110 forming the first movement space 1111. In the process of fitting the sensor unit carrier body 1310 into the column 1110, the fixing protrusion portion 1137 may come into contact with one surface of the sensor unit carrier body 1310, and the support 1134 may maintain a state torsionally deformed outward. That is, a leading end portion side of the support 1134 on which the fixing protrusion portion 1137 and the pressing movement extension portion 1136 are disposed may maintain a state of being torsionally deformed in a direction away from the first movement space 1111. As the sensor unit carrier body 1310 moves along the first direction, the support 1134 may maintain the state torsionally deformed outward, and as the fixing groove 1340 formed in the sensor unit carrier body 1310 reaches a position corresponding to the fixing protrusion portion 1137, the support 1134 may be restored to an original state from the torsionally deformed state. That is, as the leading end portion of the fixing protrusion portion 1137 is introduced into the fixing groove 1340, the pressing movement portion 1131 may move toward the sensor unit carrier body 1310, and the support 1134 may also be restored from torsional deformation.

As the leading end portion of the fixing protrusion portion 1137 is introduced into the fixing groove 1340, movement of the sensor unit carrier 130 in the first direction may be restricted. In the assembly process of the applicator assembly 1, a worker may recognize, through a sound generated by parts hitting each other or a sensation transmitted to fingertips, that the leading end portion of the fixing protrusion portion 1137 has been normally introduced into the fixing groove 1340.

The fixing portion 1130 may be provided in plurality, or a plurality of fixing protrusion portions 1137 may be provided in one fixing portion 1130, and the fixing groove 1340 may be formed to be recessed on one surface of the sensor unit carrier body 1310 in a shape and number corresponding thereto. In order to reduce friction in a process in which at least one end of the fixing protrusion portion 1137 is introduced into and discharged from the fixing groove 1340, an inclined surface may be provided on the fixing groove 1340 and/or the fixing protrusion portion 1137. The fixing groove 1340 may include not only a case of being formed to be recessed on one surface of the sensor unit carrier body 1310, but also a case of being formed to penetrate one surface of the sensor unit carrier body 1310.

Subsequently, the worker may perform a work of coupling the handle housing 120 to the body housing 110 to which the sensor unit carrier 130 is coupled. When the handle housing 120 is coupled to the body housing 110 to which the sensor unit carrier 130 is coupled, the sensor unit carrier 130 may be pressed by the handle housing 120 entering for assembly, thereby causing an arbitrary firing or causing the sensor unit carrier 130 to deviate from the initial position. However, when the assembly of the handle housing 120 is performed in a state in which the leading end portion of the fixing protrusion portion 1137 is introduced into the fixing groove 1340, an arbitrary firing of the applicator 10 or a positional deviation of the sensor unit carrier 130 may be effectively prevented. Although FIGS. 29 to 35 illustrate the push arm 1230 and fixing portion 1130 respectively provided in singular, the push arm 1230 and the fixing portion 1130 may be provided in plurality in a number corresponding to each other, and in this case, fixing stability between the body housing 110 and the sensor unit carrier 130 before coupling of the handle housing 120 may be more effectively increased. Meanwhile, since a worker may easily visually confirm positions of the push arm 1230 and the fixing portion 1130 during coupling of the body housing 110 and the handle housing 120, assembling easiness of the body housing 110 and the handle housing 120 may be more effectively improved. In a process of coupling the handle housing 120 to the body housing 110, at least one surface of the sensor unit carrier 130 may be disposed at a position in contact with an inner surface of the carrier fixing fence 1207. Since at least one end of the fixing protrusion portion 1137 is disposed in an interior of the fixing groove 1340 and coupling of the handle housing 120 and the body housing 110 is performed in a state where movement of the sensor unit carrier 130 in the first direction is restricted, one end of the sensor unit carrier 130 may be accurately introduced into an interior of the carrier fixing fence 1207. As illustrated in FIGS. 30, 31, and 33, in the process of coupling the handle housing 120 to the body housing 110 to which the sensor unit carrier 130 is coupled, the leading end portion of the push arm 1230 provided in the handle housing 120 moves to a position in contact with the pressing movement inclined surface 1133' of the pressing movement wing portion 1133. As the assembly of the handle housing 120 proceeds, the leading end portion of the push arm 1230, in a state of being in contact with the pressing movement inclined surface 1133' move in the first direction to press the pressing movement wing portion 1133, and the support 1134 is torsionally deformed outward, whereby the pressing movement portion 1131 is pushed in a direction away from the sensor unit carrier body 1310. As the pressing movement portion 1131 is pushed in the direction away from the sensor unit carrier body 1310, the leading end portion of the fixing protrusion portion 1137 may be discharged from the fixing groove 1340. As the leading end portion of the fixing protrusion portion 1137 is discharged from the fixing groove 1340, arbitrary movement restriction of the sensor unit carrier 130 by the fixing portion 1130 is released, and the sensor unit carrier 130 may maintain a state of being able to move irrespective of the fixing portion 1130. Meanwhile, although FIG. 31 illustrates a state in which a leading end of the sensor unit carrier 130 completely comes into contact with an inner surface of the handle housing 120 after completion of assembly of the applicator 10, it may include a case where, as illustrated in FIG. 30, the leading end of the sensor unit carrier 130 maintains a state of being spaced apart from the inner surface of the handle housing 120 at a predetermined interval even after completion of assembly of the applicator 10. In this case, during a process in which a user presses the handle housing 120 so that the handle housing 120 moves from the first position to the second position, a spacing distance between the leading end of the sensor unit carrier 130 and the handle housing 120 may be maintained or narrowed, and in an operation process of the applicator assembly 1, when excessive pressure is applied to the handle housing 120, a buffer may be provided against delivery of the corresponding pressure as it is to the transcutaneous sensor member 330.

As illustrated in FIG. 34, after the leading end portion of the fixing protrusion portion 1137 is discharged from the fixing groove 1340, as the push arm 1230 continuously moves in the first direction, the pressing movement wing portion 1133 comes to be positioned at a position facing the wing portion accommodation hole 1231, and the pressing movement wing portion 1133, which has passed through the barrier 1231', may have one end introduced into the wing portion accommodation hole 1231. That is, when the pressing movement wing portion 1133 passes through the barrier 1231', a leading end portion side of the support 1134 on which the fixing protrusion portion 1137 and the pressing movement extension portion 1136 are disposed is torsionally deformed in a direction becoming closer to the first movement space 1111, and accordingly, at least one end of the pressing movement wing portion 1133 may be disposed in an interior of the wing portion accommodation hole 1231.

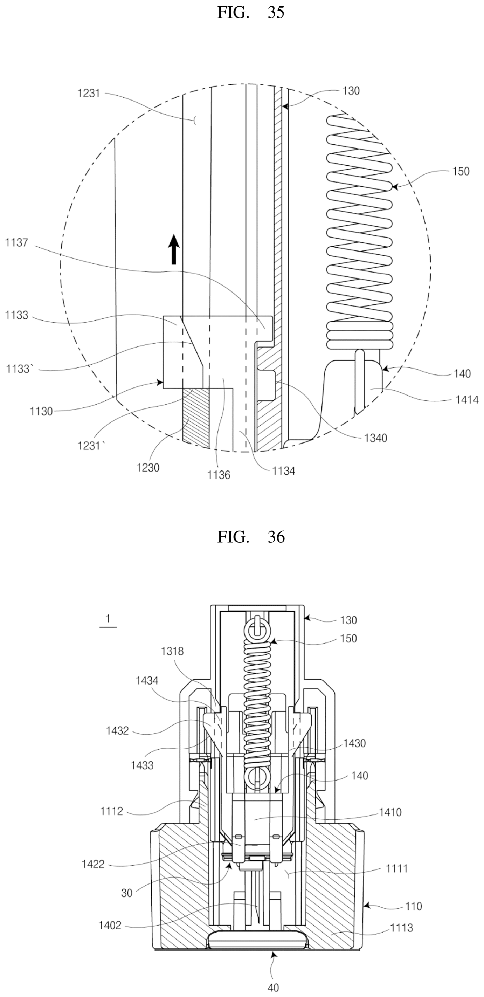

As illustrated in FIG. 35, when at least one end of the pressing movement wing portion 1133 is disposed in the interior of the wing portion accommodation hole 1231, even though the push arm 1230 moves in a direction opposite to the first direction, the barrier 1231' comes into contact with one end of the pressing movement wing portion 1133 facing the transmission unit accommodation portion 1104, thereby movement of the push arm 1230 in the direction opposite to the first direction may be restricted. That is, after insertion of the transcutaneous sensor member 330 into the subcutaneous tissue, through interaction between the push arm 1230 and the fixing portion 1130, restoration of the handle housing 120 to an initial position may be prevented, and accordingly, reuse of the applicator assembly 1 may be effectively prevented.

Meanwhile, although in the above, a case where the leading end portion of the push arm 1230 presses one end of the fixing portion 1130 so that the fixing protrusion portion 1137 is discharged from the fixing groove 1340 in a process of assembling the applicator 10 has been described as an example, it may include a case where in a firing process of the transcutaneous sensor member 330, the leading end portion of the push arm 1230 presses one end of the fixing portion 1130 so that the fixing protrusion portion 1137 is discharged from the fixing groove 1340. Unlike being illustrated in FIG. 31, even after completion of assembly of the applicator 10, the push arm 1230 may be disposed at a position in which the leading end portion thereof is adjacent to one end of the fixing portion 1130 but does not directly come into contact with the fixing portion 1130, or is in contact with one end of the fixing portion 1130 but presses the fixing portion 1130 at a degree not to discharge the fixing protrusion portion 1137 from the fixing groove 1340. That is, even when the handle housing 120 is positioned at the first position, the push arm 1230 may be disposed at a position in which a leading end portion thereof is spaced apart at a predetermined interval from one end of the fixing portion 1130, or at a position in which a leading end portion thereof comes into contact with one end of the fixing portion 1130. During a process in which a user presses the handle housing 120 so that the handle housing 120 moves from the first position to the second position, the leading end portion of the push arm 1230 may press one end of the fixing portion 1130 so that the fixing protrusion portion 1137 may be discharged from the fixing groove 1340, and accordingly, arbitrary movement restriction of the sensor unit carrier 130 by the fixing portion 1130 may be released. In this case, since a detailed process in which the fixing protrusion portion 1137 is discharged from the fixing groove 1340 corresponds to the process described above, detailed description thereof is replaced with the foregoing description.

### Exemplary embodiment of operation of needle carrier

FIGS. 36 to 38 are partial cross-sectional views sequentially illustrating an exemplary embodiment of a state in which the sensor unit carrier 130 moves from an initial position to an insertion position during an operation process of the applicator assembly 1, and FIG. 35 is a partial cross-sectional view illustrating an exemplary embodiment of a state in which the needle carrier 140 moves to a retracted position during the operation process of the applicator assembly 1.

As illustrated in FIG. 36, in a process in which the sensor unit carrier 130 moves from the initial position toward the insertion position, since the constraint surface 1434b of the needle carrier latch 1434 maintains a state of being in contact with the detent constraint surface 1318b of the sensor unit carrier detent 1318, the needle carrier 140 may maintain a state constrained to the sensor unit carrier 130 and may move along the first direction together with the sensor unit carrier 130.

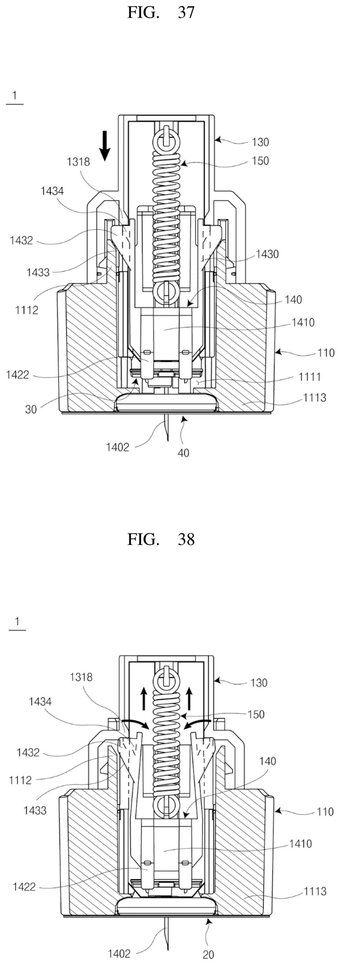

As illustrated in FIG. 37, when the sensor unit carrier 130 reaches a position adjacent to the insertion position, the trigger inclined portion 1433 of the trigger 1432 comes into contact with a leading end portion of the partition wall 1112. Meanwhile, as illustrated in FIG. 38, as the sensor unit carrier 130 continuously moves in the first direction, the needle carrier wing body 1430 is torsionally deformed toward the inside of the second movement space 1312, and accordingly, the constraint surface 1434b of the needle carrier latch 1434 and the detent constraint surface 1318b of the sensor unit carrier detent 1318 may deviate from a position of being in mutual contact. Therefore, the needle carrier latch 1434 becomes released from the constraint of the sensor unit carrier detent 1318, and the needle carrier 140 may relatively move with respect to the sensor unit carrier 130. Meanwhile, as illustrated in FIGS. 37 and 38, the release of the movement constraint of the needle carrier 140 by the sensor unit carrier 130 may be performed before the sensor unit carrier 130 reaches the insertion position, and accordingly, side effects due to excessive human body B insertion of the needle body 1402 can be effectively prevented.

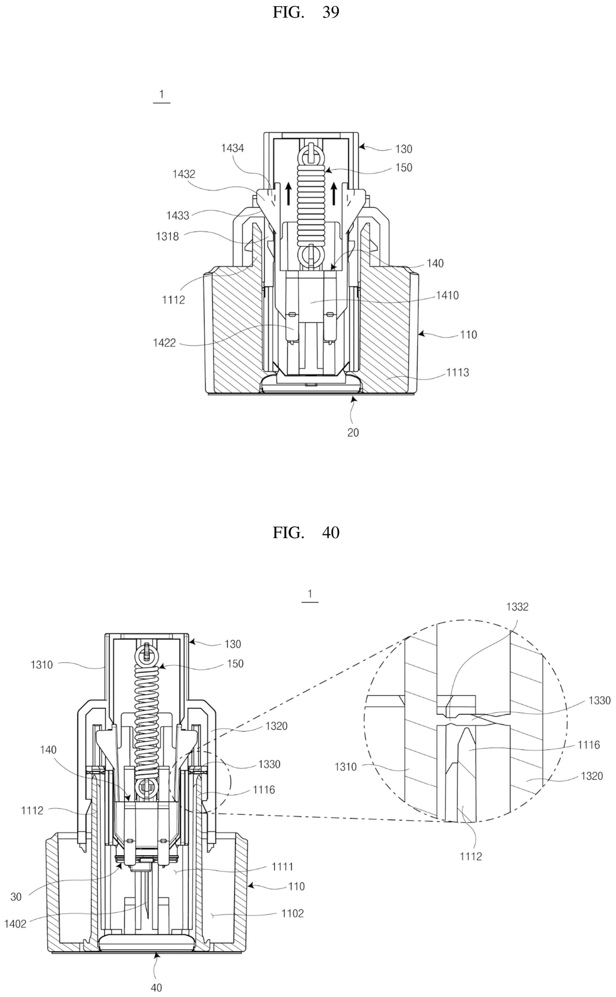

As illustrated in FIG. 39, after the constraint of the needle carrier latch 1434 by the sensor unit carrier detent 1318 is released, the needle carrier 140 may relatively move with respect to the sensor unit carrier 130, and by a driving force applied from the elastic member 150, the needle carrier 140 may move in a direction opposite to the first direction to reach the retracted position. When the needle carrier 140 reaches the retracted position, since the leading end portion of the needle body 1402 is disposed so as to be entirely accommodated in the first movement space 1111, a state in which the needle body 1402 is no longer exposed or protruded to the exterior of the applicator 10 may be maintained.

### Exemplary embodiment of action of bridge

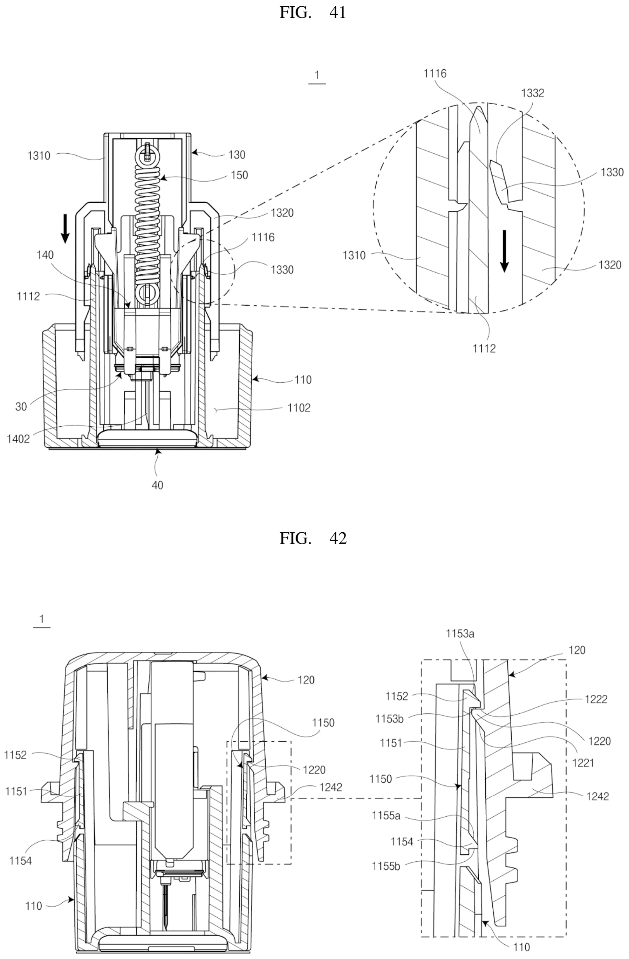

FIG. 40 is a partial cross-sectional view illustrating an exemplary embodiment of a state before the bridge 1330 is cut during an operation process of the applicator assembly 1, and FIG. 41 is a partial cross-sectional view illustrating an exemplary embodiment of a state in which the bridge 1330 is cut during the operation process of the applicator assembly 1.

FIG. 40 is a view for describing a positional relationship between the bridge 1330 and the bridge pressing portion 1116 in case where the sensor unit carrier 130 is positioned at an initial position, but as illustrated in FIG. 40, it may include not only a case where the bridge 1330 is disposed at a position spaced apart from the bridge pressing portion 1116 at a predetermined interval at the initial position, but also a case where a leading end portion of the bridge pressing portion 1116 maintains a state of coming into contact with the bridge 1330 at the initial position.

When the handle housing 120 is pressed and moved in the first direction by a user, the sensor unit carrier 130 also moves along the first direction together with the handle housing 120. Only when a force greater than fracture strength of the bridge 1330 designed in a manufacturing process is applied to the handle housing 120, the bridge 1330 may be fractured as illustrated in FIG. 37, and only when the bridge 1330 is fractured, normal firing and subcutaneous insertion of the transcutaneous sensor member 330 may be performed. Meanwhile, although FIG. 37 exemplarily describes that fracture of the bridge 1330 occurs at the vulnerable portion 1332, it may include a case where fracture of the bridge 1330 occurs at a region other than the vulnerable portion 1332.

### Exemplary embodiment of interaction between first movement restriction portion and second movement restriction portion

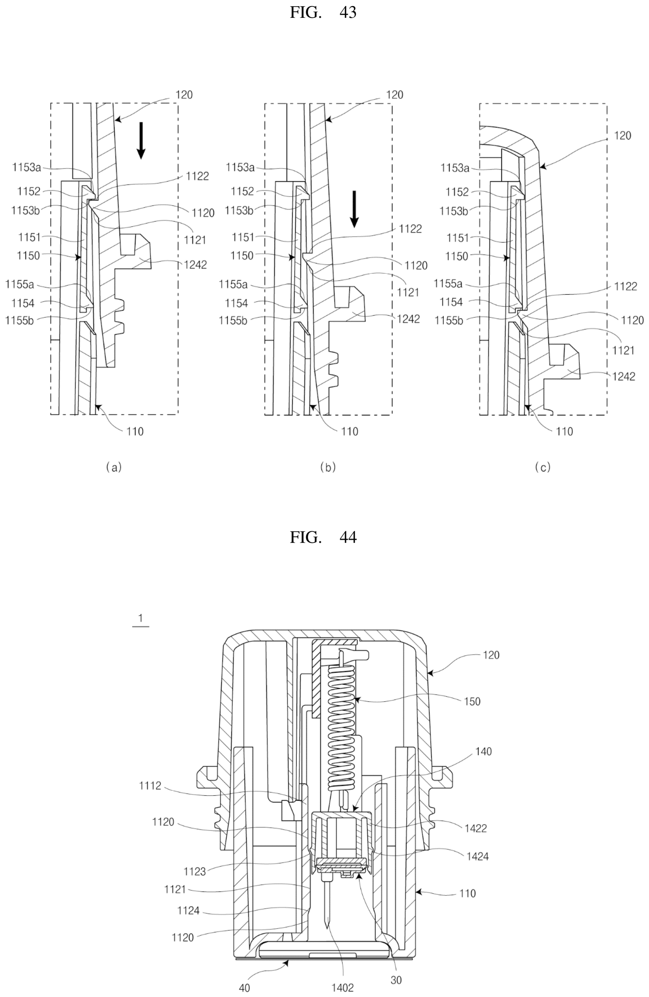

FIG. 42 is a cross-sectional view and a partial enlarged cross-sectional view for explaining an exemplary embodiment regarding a positional relationship between the first movement restriction portion 1220 and the second movement restriction portion 1150 in a state before operation of the applicator assembly 1, and FIGS. 43(a) to 41(c) are sequentially illustrated partial enlarged cross-sectional views for explaining an exemplary embodiment regarding a positional relationship between the first movement restriction portion 1220 and the second movement restriction portion 1150 during an operation process of the applicator assembly 1.

As illustrated in FIG. 42 and FIG. 43(a), in a state before operation of the applicator assembly 1, the support surface 1222 of the first movement restriction portion 1220 and the first support surface 1153b of the first movement restriction latch 1152 may be disposed to face each other. Accordingly, even when a force in a direction opposite to the first direction is applied to the handle housing 120, since the support surface 1222 of the first movement restriction portion 1220 and the first support surface 1153b of the first movement restriction latch 1152 are in contact with each other and support each other, the handle housing 120 may not further move in the direction opposite to the first direction, and thus the handle housing 120 can be effectively prevented from being arbitrarily deviated from the body housing 110.

Meanwhile, as illustrated in FIGS. 43(b) and 43(c), in a firing process of the applicator assembly 1, the inclined surface 1221 of the first movement restriction portion 1220 moves to a position adjacent to the second inclined surface 1155a of the second movement restriction latch 1154. Thereafter, in a state in which the inclined surface 1221 of the first movement restriction portion 1220 is contact with the second inclined surface 1155a of the second movement restriction latch 1154, the handle housing 120 moves in the first direction, and the other end of the movement restriction body 1151 is torsionally deformed toward the second interior space 1102, so that the first movement restriction portion 1220 passes through the second movement restriction latch 1154. After the first movement restriction portion 1220 passes through the second movement restriction latch 1154, the movement restriction body 1151 is restored to a state before torsional deformation, and the support surface 1222 of the first movement restriction portion 1220 and the second support surface 1155b of the second movement restriction latch 1154 are placed in a state facing each other. Since the movement of the handle housing 120 in a direction opposite to the first direction is restricted by the interaction between the support surface 1222 of the first movement restriction portion 1220 and the second support surface 1155b of the second movement restriction latch 1154, reuse of the applicator 10 after firing of the transcutaneous sensor member 330 can be strictly restricted.

### Exemplary embodiment of action of grip arm protrusion portion

FIGS. 44 to 46 are sequentially illustrated cross-sectional views for explaining an exemplary embodiment of pressing and pressing release of the grip arm protrusion portion 1424 during an operation process of the applicator assembly 1.

FIG. 44 is a view illustrating a state before operation of the applicator assembly 1, and the grip arm protrusion portion 1424 may be disposed in a state of not having passed through the first step inclined surface 1123. That is, since the grip arm protrusion portion 1424 maintains a state of not being pressed by the step portion 1121, the grip arm 1422 may grip the sensor unit 30 in a somewhat loose state. Meanwhile, unlike FIG. 44, the grip arm protrusion portion 1424 may be disposed in a state of having passed through the first step inclined surface 1123 in the state before operation of the applicator assembly 1, and in this case, the grip arm protrusion portion 1424 is pressed by the step portion 1121 so that the grip arm 1422 may maintain a state of gripping the sensor unit 30 firmly.

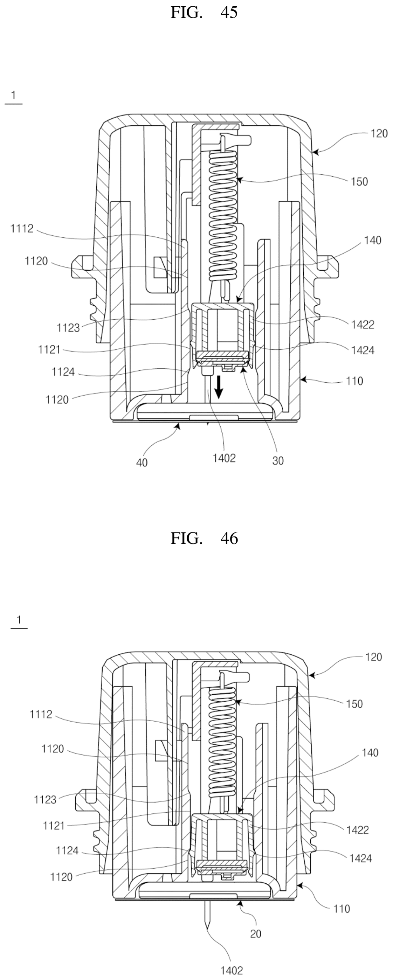

When the handle housing 120 is pressed in the first direction by a user, as illustrated in FIG. 45, the grip arm protrusion portion 1424 maintains a state of being pressed by the step portion 1121 and moves in the first direction, and in this process, the sensor unit 30 maintains a state of being firmly gripped by the grip arm 1422 and may move toward the transmission unit 40.

Thereafter, as illustrated in FIG. 46, the grip arm protrusion portion 1424 passes through the second step inclined surface 1124 so that the pressing of the grip arm protrusion portion 1424 by the step portion 1121 may be released, and the grip arm 1422 may grip the sensor unit 30 in a somewhat loose state so as to deliver the sensor unit 30 to the transmission unit 40. In the insertion process of the transcutaneous sensor member 330 into the subcutaneous tissue, since the sensor unit 30 continuously receives the force in the first direction by the sensor unit housing 310, even if the grip arm 1422 grips the sensor unit 30 in a somewhat loose state to deliver the sensor unit 30 to the transmission unit 40, the sensor unit 30 may be accurately delivered to the seating groove 412. Meanwhile, since the grip arm 1422 grips the sensor unit 30 in a somewhat loose state to deliver the sensor unit 30 to the seating groove 412 of the transmission unit 40, an operational error that occurs in which the sensor unit 30 is brought along with the needle carrier 140, in a process in which the needle carrier 140 returns to a retracted position, can be effectively prevented.

### Exemplary embodiment of operation of transmission unit support portion

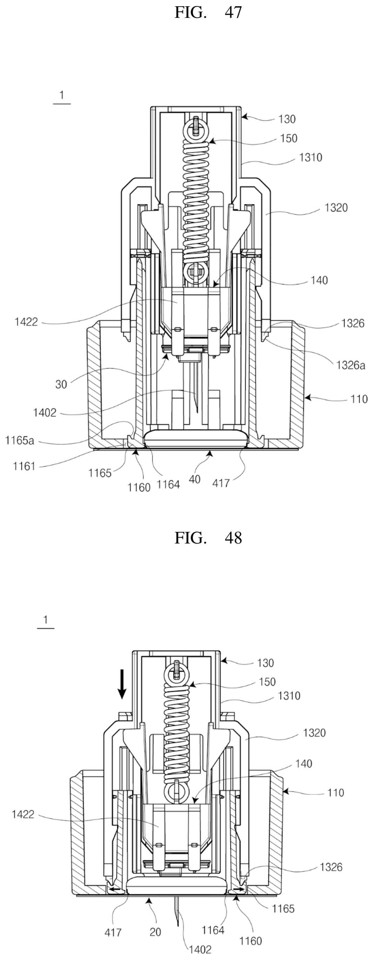

FIGS. 47 and 48 are sequentially illustrated cross-sectional views for explaining an exemplary embodiment of movement constraint and movement constraint release of the transmission unit 40 by the transmission unit support portion 1160 during an operation process of the applicator assembly 1.

As illustrated in FIG. 47, in a state before operation of the applicator assembly 1, a leading end portion of the catching portion 1164 provided in the transmission unit support hook 1162 may be disposed in an interior of the transmission unit housing groove 417 of the transmission unit housing 410 so as to effectively prevent the transmission unit 40 from being arbitrarily deviated from the transmission unit accommodation portion 1104. In this case, the extension arm pushing portion 1326 provided in the sensor unit carrier 130 may maintain a state spaced apart from the pushed portion 1165 of the transmission unit support hook 1162. Since the transmission unit 40 is prevented by the transmission unit support hook 1162 from being arbitrarily deviated from the transmission unit accommodation portion 1104 before operation of the applicator assembly 1 or in the insertion process of the transcutaneous sensor member 330 into the subcutaneous tissue, the economic efficiency and operational accuracy of the applicator assembly 1 can be effectively improved.

Meanwhile, as illustrated in FIG. 48, when the applicator assembly 1 operates and the sensor unit carrier 130 moves to an insertion position, the extension arm pushing portion 1326 presses the inclined surface 1165a formed on the pushed portion 1165 so that the transmission unit support hook 1162 moves while being torsionally deformed in a direction away from the transmission unit 40, and accordingly, the leading end portion of the catching portion 1164 may be deviated from the transmission unit housing groove 417, thereby releasing the movement constraint of the transmission unit 40 by the transmission unit support hook 1162. The transmission unit support portion 1160, through interaction with the extension arm pushing portion 1326, releases the movement constraint of the transmission unit 40 by the transmission unit support hook 1162 in the insertion process of the transcutaneous sensor member 330 into the subcutaneous tissue, thereby effectively preventing a phenomenon in which the wearable unit 20 is brought along with the applicator 10 in a process of separating the wearable unit 20 from the applicator 10 after completion of the insertion of the transcutaneous sensor member 330 into the subcutaneous tissue.

Although the present invention has been described in detail through the embodiments above, other forms of embodiments are also possible. Therefore, the technical teachings and scope of the claims described below are not limited to the embodiments.

### [Description of Reference Numerals]

| | | | |
|---|---|---|---|
| 1: | Applicator assembly | 5: | External terminal |
| 10: | Applicator | 20: | Wearable unit |
| 30: | Sensor unit | 40: | Transmission unit |
| 50: | Cap | 110: | Body housing |
| 120: | Handle housing | 130: | Sensor unit carrier |
| 140: | Needle carrier | 150: | Elastic member |

## Claims

1. An applicator, comprising:
a body housing in which a movement space is formed along a first direction;
a sensor unit carrier provided to be movable along the movement space and comprising a fixing groove formed to be recessed on one surface thereof;
a handle housing coupled to the body housing and configured such that at least a portion thereof is in contact with the sensor unit carrier to move together with the sensor unit carrier; and
a fixing portion disposed on the body housing to move between a fixing position in which one end portion thereof is inserted into the fixing groove and a release position in which the one end portion is disengaged from the fixing groove.

2. The applicator of claim 1, wherein movement of the sensor unit carrier is restricted by the fixing portion positioned at the fixing position, and the movement restriction of the sensor unit carrier is released as the fixing portion moves to the release position.

3. The applicator of claim 1, wherein the fixing portion moves from the fixing position to the release position in a process of coupling the handle housing to the body housing.

4. The applicator of claim 1, wherein the fixing portion comprises:
a support disposed to extend along a direction parallel to the first direction and provided to be torsionally deformable;
a fixing protrusion portion provided to protrude toward the movement space from one surface of the support facing the movement space; and
a pressing movement portion connected to the other surface opposite to the one surface of the support facing the fixing protrusion portion.

5. The applicator of claim 4, wherein the handle housing comprises a push arm extending toward the pressing movement portion from an inner one end of the handle housing, and
wherein, during coupling of the handle housing to the body housing, a leading end portion of the push arm presses the pressing movement portion so that the fixing portion positioned at the fixing position transitionally moves to the release position.

6. The applicator of claim 5, wherein the pressing movement portion comprises a pressing movement wing portion provided at a position corresponding to the leading end portion of the push arm, and
wherein the pressing movement wing portion is provided with a pressing movement inclined surface having an inclination becoming adjacent to the sensor unit carrier along the first direction.

7. The applicator of claim 6, wherein the push arm comprises a wing portion accommodation hole formed to extend along the first direction in a shape penetrating the push arm, and
wherein after the fixing portion moves to the release position, at least one end of the pressing movement wing portion is disposed in an interior of the wing portion accommodation hole.

8. The applicator of claim 7, wherein the push arm further comprises a barrier provided on the leading end portion of the push arm adjacent to the fixing portion so as to close one end of the wing portion accommodation hole, and
wherein one end of the pressing movement wing portion introduced into the interior of the wing portion accommodation hole contacts with the barrier, thereby restricting a return to an initial position of the handle housing moved in the first direction.

9. The applicator of claim 8, wherein as the leading end portion of the push arm presses the pressing movement inclined surface in the first direction, the support is torsionally deformed in a direction in which one end of the support to which the fixing protrusion portion and the pressing movement portion are connected moves away from the movement space.

10. The applicator of claim 4, wherein the body housing further comprises a column disposed to partition the movement space in an interior of the body housing, and
wherein the support is formed by incision of one side wall of the column.

11. The applicator of claim 10, wherein the support is configured to be divided by a first incision groove and a second incision groove formed adjacent so as to at least partially incise the one side wall of the column.

12. An applicator assembly, comprising:
a body housing in which a movement space is formed along a first direction, and in which a transmission unit is separably fixed and disposed at one end thereof;
a sensor unit carrier comprising a fixing groove formed to be recessed on one side surface thereof, and configured to move in the movement space from an initial position to an insertion position together with a sensor unit including a transcutaneous sensor member to deliver the sensor unit to the transmission unit;
a handle housing coupled to the body housing so as to be relatively movable with respect to the body housing and configured to move from a first position to a second position together with the sensor unit carrier by pressing of a user; and
a fixing portion disposed on the body housing so as to move from a fixing position in which one end portion thereof is inserted into the fixing groove to a release position in which the one end portion is disengaged from the fixing groove, during a process of coupling the handle housing to the body housing or during a process in which the handle housing moves from the first position to the second position.

13. The applicator assembly of claim 12, wherein the handle housing comprises a push arm extending toward the fixing portion from an inner one end of the handle housing, and
wherein a leading end portion of the push arm presses the fixing portion so that the fixing portion positioned at the fixing position moves to the release position.

14. The applicator assembly of claim 13, wherein when the handle housing is positioned at the first position, the leading end portion of the push arm is positioned at a position spaced apart from one end of the fixing portion or positioned at a position in contact with one end of the fixing portion, and
wherein as the handle housing moves to the second position, the leading end portion of the push arm presses one end of the fixing portion so that the fixing portion moves to the release position.

15. The applicator assembly of claim 13, wherein the first position is a position of the handle housing when the sensor unit carrier is positioned at the initial position after completion of assembly of the applicator assembly, and
wherein the second position is a position of the handle housing after the sensor unit carrier moves to the insertion position and the sensor unit is delivered to the transmission unit.
